# EUROPEAN PATENT APPLICATION

(11) **EP 4 790 133 A1**
(43) Date of publication of application: **12.08.2026**
(21) Application number: 24874809.7
(22) Date of filing: 02.10.2024
(51) Int. Cl.: C12N 5/0775, C12M 1/34, C12Q 1/02, G06T 7/00, G06V 20/69

(54) **METHOD FOR MANAGING QUALITY OF STEM CELLS BY IMAGE ANALYSIS USING AI**

(30) Priority: 02.10.2023 JP 2023171649
(71) Applicant: PUREC CO., LTD., Izumo-shi, Shimane 693-0021 (JP); NATIONAL UNIVERSITY CORPORATION TOKAI NATIONAL HIGHER EDUCATION AND RESEARCH SYSTEM, Nagoya-shi, Aichi 464-8601 (JP)
(72) Inventor: KATO Ryuji, Nagoya-shi, Aichi 464-8601 (JP); KANIE Kei, Nagoya-shi Aichi 464-8601 (JP); TAKEMOTO Yuto, Nagoya-shi Aichi 464-8601 (JP); IYOKU MATSUZAKI Yumi, Izumo-shi, Shimane 693-0021 (JP); SUYAMA Takashi, Izumo-shi, Shimane 693-0021 (JP)
(74) Representative: J A Kemp LLP
(86) International application number: PCT/JP2024/080176
(87) International publication number: WO 2025/075210

(57) **Abstract**

Provided is a reference standard for evaluating or determining the quality of cells of interest, the reference standard comprising a rapidly expanding mesenchymal stem cell population characterized by using, as an indicator, the positivity or negativity of at least one marker selected from LNGFR (CD271) and Thy-1 (CD90).

## Description

### [TECHNICAL FIELD]

The present invention relates to image analysis, particularly to a method for quality control of stem cells by image analysis using AI.

### [BACKGROUND ART]

Regenerative medicine is a medical treatment that regenerates organs that have fallen into dysfunction. Examples that have already been put into practical use include heart transplantation, liver transplantation, kidney transplantation, and hematopoietic stem cell transplantation.

Three types of stem cells are expected to be applied in regenerative medicine: embryonic stem cells (ES cells) obtained from fertilized eggs; induced pluripotent stem cells (iPS cells) obtained by introducing the Yamanaka four factors into dermal fibroblasts; and somatic stem cells present in tissues and organs within the body. Currently, research and development for drug discovery applications and clinical applications using these cells is actively conducted worldwide.

Stem cells exist in all tissues throughout the body, and mesenchymal stem cells (MSCs) are one type of such cells. MSCs are mainly present in bone marrow and are involved in the regeneration of bone and cartilage. In particular, in recent years, cell products using MSCs have been approved as regenerative medicine products, and the possibility of mass-producing cells using allogeneic cells while reducing costs and responding to large markets has expanded. As a result, the industrialization of regenerative medicine is rapidly accelerating.

The present inventors developed a technique for directly isolating human MSCs from bone marrow using two antibodies, LNGFR (CD271) and Thy1 (CD90), together with a cell sorter. Furthermore, they succeeded in sorting out and obtaining ultra-high-purity human MSCs, as a cell population with extremely high proliferation and differentiation potentials. Such ultra-high-purity human MSCs were named REC (Rapidly Expanding Cell). It was also revealed that REC does not cause side effects such as pulmonary embolism even when more than 100 times the number of cells compared to MSCs cultured using conventional methods are transplanted (Patent Literatures 1-4, Non-Patent Literature 1).

However, in current regenerative medicine, the price of a single regenerative medicine product under clinical research is estimated to be several million to several tens of millions of yen. This makes it difficult to deliver such treatments to many patients and poses a challenge of placing a burden on healthcare finances.

The primary reason regenerative medicine is expensive is the inefficiency of the cell manufacturing process. Currently, all cells for regenerative medicine are handmade, produced one by one, and can only be manufactured by highly skilled experts. A particularly significant issue is the lack of cell quality control technology. Even in the world's leading research facilities, hospitals, or cell banks, cell quality evaluation inevitably depends on the expert judgment of skilled personnel.

Meanwhile, the present inventors have developed a technology that integrates image processing technology with AI (artificial intelligence) and machine learning, i.e., cell morphological information analysis, to non-destructively, in real time, at low cost, and quantitatively inspect and predict the quality of cells during culture for various types of human cells used in cell therapy (Patent Literatures 5-8, Non-Patent Literature 2).

Cell morphological information analysis is a technology that integrates image processing, data mining, and AI analysis technologies to predict biological evaluation results (quality) of cells solely from images. Using cell morphological information analysis technology, information related to cell quality, which was previously obtainable only by staining after long-term culture, can now be detected as early as three hours after seeding.

However, the development of the above cell morphological information analysis technology requires a large amount of high-quality data (images of functional stem cells and quality data). Even when using commercially available cells, the cost is extremely high, and conventionally, analysis has been limited to data from only 10-20 cells. Furthermore, commercially available cells have unstable quality between lots and are mixtures of cells with various properties, leaving issues regarding cell suitability.

### [CITATION LIST]

### [PATENT LITERATURE]

[Patent Literature 1] Japanese Patent No. 6463029
[Patent Literature 2] Japanese Patent No. 6850944
[Patent Literature 3] Japanese Patent No. 6932390
[Patent Literature 4] WO2021/145002
[Patent Literature 5] Japanese Patent Application Publication No. 2016-189701
[Patent Literature 6] Japanese Patent Application Publication No. 2016-189702
[Patent Literature 7] Japanese Patent Application Publication No. 2016-191565
[Patent Literature 8] Japanese Patent No. 6721895

### [NON-PATENT LITERATURE]

[Non-Patent Literature 1] Mabuchi Y et al., "LNGFR+ Thy-1+ Vcam-1hi+ cells reveal functionally distinct subpopulations in mesenchymal stem cells". Stem Cell Reports 1(2): 152-165, 2013
[Non-Patent Literature 2] NEDO FY 2018-2019 Final Report
   Next-Generation Artificial Intelligence and Robot Core Technology Development / Next-Generation Artificial Intelligence Technology Field / Research on Advanced Quality Inspection of High-Purity Mesenchymal Stem Cells Using AI

### [SUMMARY OF INVENTION]

### [TECHNICAL PROBLEM]

In view of the above circumstances, there has been a demand for the development of cells suitable for cell morphological information analysis technology.

### [SOLUTION TO PROBLEM]

As a result of intensive studies to solve the above problems, the present inventors found that REC is useful for cell morphological information analysis technology, and thus completed the present invention.

That is, the present invention is as follows.
[1] A reference standard for evaluating or determining the quality of cells of interest, the reference standard comprising a rapidly expanding mesenchymal stem cell population characterized by using, as an indicator, the positivity or negativity of at least one marker selected from LNGFR (CD271) and Thy-1 (CD90).
[2] The reference standard according to item [1], wherein characteristics of the cell population are selected from any of the following information (a) to (d):
   (a) at least one piece of cell information selected from the group consisting of cell size, degree of spreading, contour complexity, pattern, and brightness of individual cells within the cell population, and image information thereof;
   (b) information obtained by integrating the cell information of (a) across multiple cells within the cell population and statistically processing it;
   (c) combination information obtained by acquiring the information of (b) for multiple cell populations; and
   (d) information of (a) to (c) acquired over time.
[3] The reference standard according to item [2], wherein the statistically processed information is at least one selected from the group consisting of mean value, standard deviation, and distribution shape.
[4] The reference standard according to item [1], wherein the quality is at least one selected from the group consisting of cell proliferation rate, cell recovery number, degree of undifferentiation, degree of differentiation, amount of produced factors, and cell heterogeneity.
[5] The reference standard according to item [1], wherein the cells of interest are stem cells.
[6] The reference standard according to item [5], wherein the stem cells of interest are mesenchymal stem cells.
[7] A method for providing information for evaluating the quality of a stem cell population of interest, the method comprising the steps of:
   (a) characterizing individual cells of a rapidly expanding mesenchymal stem cell population by using, as an indicator, the positivity or negativity of at least one marker selected from LNGFR (CD271) and Thy-1 (CD90), and acquiring image data (reference image information) of the individual cells, or acquiring parameter information (reference parameter information) representing characteristics of the cell population from the reference image information;
   (b) acquiring image data (image information of interest) of individual cells of a stem cell population of interest provided by a user, or acquiring parameter information (parameter information of interest) representing characteristics of the stem cell population of interest from the image information of interest;
   (c) comparing the image information of interest or parameter information of interest with the reference image information or reference parameter information; and
   (d) providing to the user all or part of the information obtained by the comparison for evaluating the quality of the stem cell population of interest.
[8] A method for evaluating the quality of a stem cell population of interest, the method comprising the steps of:
   (a) characterizing individual cells of a rapidly expanding mesenchymal stem cell population by using, as an indicator, the positivity or negativity of at least one marker selected from LNGFR (CD271) and Thy-1 (CD90), and acquiring image data (reference image information) of the individual cells, or acquiring parameter information (reference parameter information) representing characteristics of the cell population from the reference image information;
   (b) acquiring image data (image information of interest) of individual cells of a stem cell population of interest provided by a user, or acquiring parameter information (parameter information of interest) representing characteristics of the stem cell population of interest from the image information of interest;
   (c) comparing the image information of interest or parameter information of interest with the reference image information or reference parameter information; and
   (d) evaluating the quality of stem cells of interest by using, as an indicator, all or part of the information obtained by the comparison.
[9] A system for providing information for evaluating the quality of a stem cell population of interest, the system comprising:
   (a) a unit configured to characterize individual cells of a rapidly expanding mesenchymal stem cell population by using, as an indicator, the positivity or negativity of at least one marker selected from LNGFR (CD271) and Thy-1 (CD90), and acquire image data (reference image information) of the individual cells, or acquire parameter information (reference parameter information) representing characteristics of the cell population from the reference image information;
   (b) a unit configured to acquire image data (image information of interest) of individual cells of a stem cell population of interest provided by a user, or acquire parameter information (parameter information of interest) representing characteristics of the stem cell population of interest from the image information of interest;
   (c) a unit configured to compare the image information of interest or parameter information of interest with the reference image information or reference parameter information; and
   (d) a unit configured to provide to the user all or part of the information obtained by the comparison for evaluating the quality of the stem cell population of interest.
[10] A system for evaluating the quality of a stem cell population of interest, the system comprising:
   (a) a unit configured to characterize individual cells of a rapidly expanding mesenchymal stem cell population by using, as an indicator, the positivity or negativity of at least one marker selected from LNGFR (CD271) and Thy-1 (CD90), and acquire image data (reference image information) of the individual cells, or acquire parameter information (reference parameter information) representing characteristics of the cell population from the reference image information;
   (b) a unit configured to acquire image data (image information of interest) of individual cells of a stem cell population of interest provided by a user, or acquire parameter information (parameter information of interest) representing characteristics of the stem cell population of interest from the image information of interest;
   (c) a unit configured to compare the image information of interest or parameter information of interest with the reference image information or reference parameter information; and
   (d) a unit configured to evaluate the quality of stem cells of interest by using, as an indicator, all or part of the information obtained by the comparison.
[11] The system according to item [9] or [10], wherein characteristics of the cell population are selected from any of the following information (a) to (d):
   (a) at least one piece of cell information selected from the group consisting of cell size, degree of spreading, contour complexity, pattern, and brightness of individual cells within the cell population, and image information thereof;
   (b) information obtained by integrating the cell information of (a) across multiple cells within the cell population and statistically processing it;
   (c) combination information obtained by acquiring the information of (b) for multiple cell populations; and
   (d) information of (a) to (c) acquired over time.
[12] The system according to item [11], wherein the statistically processed information is at least one selected from the group consisting of mean value, standard deviation, and distribution shape.
[13] The system according to item [9] or [10], wherein the quality is at least one selected from the group consisting of cell proliferation rate, cell recovery number, degree of undifferentiation, degree of differentiation, amount of produced factors, and cell heterogeneity.
[14] The system according to item [9] or [10], wherein the stem cells of interest are mesenchymal stem cells.
[15] A program for providing information for evaluating the quality of a stem cell population of interest, the program causing a computer to function as:
   (a) a unit configured to characterize individual cells of a rapidly expanding mesenchymal stem cell population by using, as an indicator, the positivity or negativity of at least one marker selected from LNGFR (CD271) and Thy-1 (CD90), and acquire image data (reference image information) of the individual cells, or acquire parameter information (reference parameter information) representing characteristics of the cell population from the reference image information;
   (b) a unit configured to acquire image data (image information of interest) of individual cells of a stem cell population of interest provided by a user, or acquire parameter information (parameter information of interest) representing characteristics of the stem cell population of interest from the image information of interest;
   (c) a unit configured to compare the image information of interest or parameter information of interest with the reference image information or reference parameter information; and
   (d) a unit configured to provide to the user all or part of the information obtained by the comparison for evaluating the quality of the stem cell population of interest.
[16] A program for evaluating the quality of a stem cell population of interest, the program causing a computer to function as:
   (a) a unit configured to characterize individual cells of a rapidly expanding mesenchymal stem cell population by using, as an indicator, the positivity or negativity of at least one marker selected from LNGFR (CD271) and Thy-1 (CD90), and acquire image data (reference image information) of the individual cells, or acquire parameter information (reference parameter information) representing characteristics of the cell population from the reference image information;
   (b) a unit configured to acquire image data (image information of interest) of individual cells of a stem cell population of interest provided by a user, or acquire parameter information (parameter information of interest) representing characteristics of the stem cell population of interest from the image information of interest;
   (c) a unit configured to compare the image information of interest or parameter information of interest with the reference image information or reference parameter information; and
   (d) a unit configured to evaluate the quality of stem cells of interest by using, as an indicator, all or part of the information obtained by the comparison.
[17] The program according to item [15] or [16], wherein characteristics of the cell population are selected from any of the following information (a) to (d):
   (a) at least one piece of cell information selected from the group consisting of cell size, degree of spreading, contour complexity, pattern, and brightness of individual cells within the cell population, and image information thereof;
   (b) information obtained by integrating the cell information of (a) across multiple cells within the cell population and statistically processing it;
   (c) combination information obtained by acquiring the information of (b) for multiple cell populations; and
   (d) information of (a) to (c) acquired over time.
[18] The program according to item [17], wherein the statistically processed information is at least one selected from the group consisting of mean value, standard deviation, and distribution shape.
[19] The program according to item [15] or [16], wherein the quality is at least one selected from the group consisting of cell proliferation rate, cell recovery number, degree of undifferentiation, degree of differentiation, amount of produced factors, and cell heterogeneity.
[20] The program according to item [15] or [16], wherein the stem cells of interest are mesenchymal stem cells.
[21] A computer-readable recording medium storing the program according to item [15] or [16].
[22] An image processing method for rapidly expanding mesenchymal stem cells, the method comprising the steps of:
   (a) acquiring image data of the reference standard according to item [1]; and
   (b) removing noise from the acquired image data and/or normalizing the image data.
[23] A method for providing image data processed by the method according to item [22] for machine learning by a computer.
[24] A method for predicting the quality of cells of interest by comparing image data processed by the method according to item [22] with image data of the cells of interest.

### [EFFECTS OF INVENTION]

The present invention provides a reference standard for evaluating or determining the quality of cells of interest, the reference standard comprising a rapidly expanding mesenchymal stem cell population. The reference standard of the present invention achieves efficiency and cost reduction in quality control during the manufacture of stem cells as cell therapeutics, thereby enabling reduction of stem cell pharmaceutical manufacturing costs.

### BRIEF DESCRIPTION OF DRAWINGS

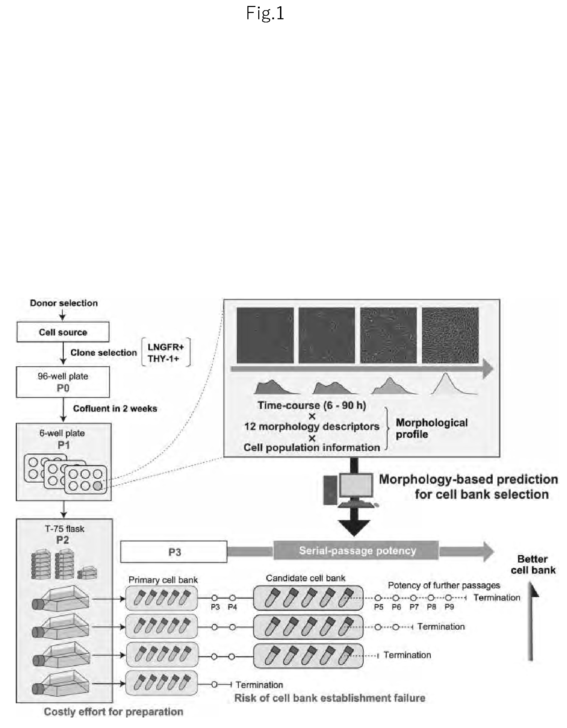

[Fig. 1] Fig. 1 is a conceptual diagram of predicting serial passage potency using morphological profiles for selecting a highly potent cell bank. Target REC was sorted out from MSCs through clone selection steps (P1 and P2). At P3, the cells were cryopreserved to form a primary cell bank for preparing initial passage cells for further experiments. A serial passage test was conducted from P3 to the passage limit. For the manufacture of practical cell-based therapeutic products, candidate cell banks are formed during such serial passage. However, there is a risk that, for example, cells may exhibit unexpected growth arrest, resulting in failure to establish a cell bank. In addition, although proliferation potential may decrease, it is expected that a candidate cell bank with lower risk, i.e., a higher-quality candidate cell bank, can be formed, from which banked cells with higher activity and higher potential can be obtained. Such serial passage potency was predicted from the morphological profile of cell images at P2. The morphological profile consists of time-course data × 12 morphological descriptors × cell population information (mean value and SD).
[Fig. 2] Fig. 2 is a diagram showing the profiles of 15 REC clones. Fig. 2a shows results of serial passage tests of REC. Fig. 2b shows representative morphology of REC. Fig. 2c shows growth profiles of REC. Fig. 2d shows the correlation between proliferation rate and limit number of serial passages. R² indicates the coefficient of determination.

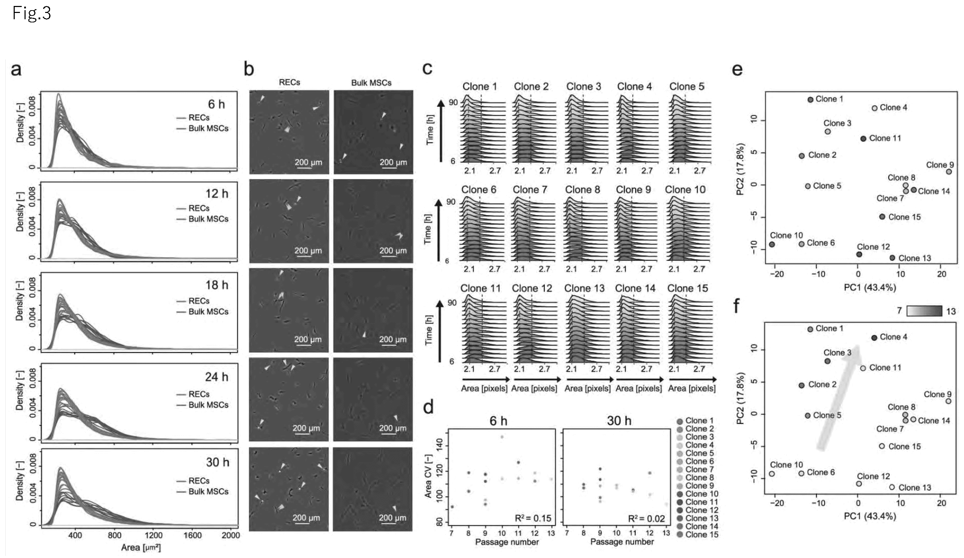

[Fig. 3] Fig. 3a shows comparison of size distribution and its time-course changes between 15 REC clones and conventionally processed bulk MSCs (cMSC, 9 lots), and morphological characterization of the 15 RECs. Only adherent and spreading cells were counted. The dashed vertical line represents the mean cell size. Fig. 3b shows representative time-course images of REC and bulk MSCs. Yellow and blue arrows indicate proliferating cells and recently proliferated cells, respectively. Fig. 3c shows changes in size distribution and time-course among the 15 clones. Fig. 3d shows the correlation between "SD of area" and passage number; R² is the coefficient of determination. Figs. 3e and 3f show PCA plots of 15 RECs profiled using 24 morphological descriptors. Fig. 3e is a PCA plot with clone color labels. Fig. 3f is a PCA plot colored by serial passage potency heat map.

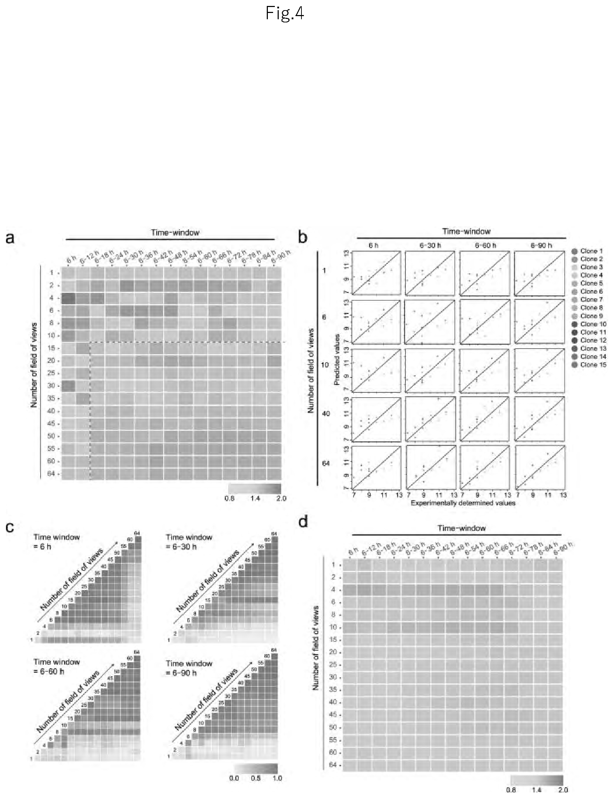

[Fig. 4] Fig. 4 is a diagram showing comprehensive evaluation of data utilization effects and performance of a serial passage prediction model. Fig. 4a shows evaluation of data utilization effects using LASSO. Row values represent the number of FOVs used, and column values represent the size of the time window used for training data. The heat map shows RMSE. RMSE < 1.0 is considered a model with good performance. Fig. 4b is a scatter plot visualizing the performance of the serial passage prediction model, where each dot represents one clone. Fig. 4c shows comparison of model structures between two pairs of constructed models, selecting time windows of 6, 6-30, 6-60, and 6-90 hours. The heat map shows correlation coefficients among all weights of all selected morphological descriptors in the models. The correlation coefficients are high when the combinations of descriptors used are similar. Fig. 4d shows evaluation of data utilization effects using RF. Rows represent the number of FOVs used, and columns represent the time window size used for training data. The heat map shows RMSE. RMSE < 1.0 is considered a model with good performance.
[Fig. 5] Fig. 5 shows robustness of the serial passage prediction model against data variation included by bootstrap FOV selection (50 iterations), and evaluation of its data utilization effects.
[Fig. 6] Fig. 6 is a block diagram of the system of the present invention.
[Fig. 7] Fig. 7 is a flowchart showing the operation of the system of the present invention.
[Fig. 8] Fig. 8 is a diagram showing information extracted from image data of stem cells. Red indicates REC clone MSCs, showing that the information is homogeneous and well-organized. The horizontal axis represents quantified indices. The mountain-shaped curves represent histograms of cell populations consisting of several thousand cells.
[Fig. 9] Fig. 9 is a diagram showing information extracted from image data of stem cells. Information extracted from REC image data is shown to be well-organized across multiple indices. Blue dots represent bulk MSCs, which are scattered in various locations, whereas red dots represent REC clones, forming a clean elliptical shape as a whole.
[Fig. 10] Fig. 10 is a diagram showing information extracted from image data of stem cells. The left panel represents REC clones, and the right panel represents bulk (commercially available MSC) stem cell populations. The vertical axis represents time. REC clones maintain a single distribution over time. Bulk MSCs show two peaks in the early culture stage, which merge or mix over time, indicating that the cell information is convoluted.
[Fig. 11] Fig. 11 is a diagram showing the results of cell sorting using a flow cytometer. Through the sorting process using a flow cytometer, the population becomes clonal, resulting in various clones. This indicates that clones previously discarded as non-REC may also be utilized as cells with certain characteristics for machine learning.
[Fig. 12] Fig. 12 is a diagram showing proliferation rate (upper panel), adipogenic differentiation (middle panel), and osteogenic differentiation (lower panel) of each MSC.
[Fig. 13] Fig. 13 is a diagram showing adipogenic differentiation prediction models for each MSC. REC clone data show high accuracy, whereas bulk data show low accuracy.
[Fig. 14] Fig. 14 is a diagram showing the results of predicting bulk data using the clone model.
[Fig. 15] Fig. 15 is a diagram showing the integration of cell morphological information.
[Fig. 16] Fig. 16 is a diagram showing the integration of cell morphological information.
[Fig. 17] Fig. 17 is a diagram showing morphological profiles of cell populations.
[Fig. 18] Fig. 18 is a diagram showing proliferation profiles of bulk MSCs.

### [DESCRIPTION OF EMBODIMENTS]

### 1. Overview

The present invention relates to a reference standard for evaluating or determining the quality of cells of interest, the reference standard comprising a rapidly expanding mesenchymal stem cell (REC) population.

In the present invention, cell image information analysis was performed for each REC clone, and based on the results obtained by comparing the analysis with actual culture data, a learning model was constructed using the data of each REC clone, thereby completing a quality evaluation system.

More than 100 clones were used for cell image information analysis, including clones at commercialization level, clones in which deviations were observed, clones derived from different donor lots, and clones cultured under altered culture conditions. Detailed culture process data were also organized as metadata together with image data and incorporated into a database. Furthermore, experimental results regarding osteogenic differentiation, adipogenic differentiation potential, and proliferation potential for each clone were added to the database.

In one embodiment of the present invention, while utilizing AI, three types of nonlinear regression models were selected in addition to conventional machine learning models such as linear regression, and their source code and computational environment were developed. In the present invention, learning models using REC clone data were validated, and by referencing and comparing REC data, a method for evaluating the quality of other mesenchymal stem cells was established. For example, it was found that the cell recovery rate of REC after five days could be predicted with high accuracy from data obtained during as little as two days of proliferation culture, and that deviations in differentiation potential one month later could be predicted from images obtained during proliferation culture. Furthermore, using the quality database comprehensively validated with REC cells, the heterogeneity of MSCs from other companies was quantitatively evaluated based on REC data, and it was demonstrated that the quality of such MSCs could be predicted with nearly the same performance.

### 2. Reference Standard for Evaluating or Determining the Quality of Cells of Interest

The reference standard used in the present invention is a rapidly expanding mesenchymal stem cell population characterized by using, as an indicator, the positivity or negativity of at least one marker selected from LNGFR (CD271) and Thy-1 (CD90).

Rapidly expanding mesenchymal stem cells in which at least one of LNGFR (CD271) and Thy-1 (CD90) is positive or negative may be obtained, for example, according to the method described in WO2009/31678.

An overview of this method is as follows.

First, mesenchymal stem cells are highly enriched by sorting out cell fractions in which at least one of CD271 and CD90 is positive (CD271+/CD90+) from a cell population containing human mesenchymal stem cells. If the cell population containing human mesenchymal stem cells contains hematopoietic cells, a step of sorting out cells that are double-negative for CD45 and CD235a (CD45-CD235a-) can be added to sort out non-hematopoietic cells.

The cell population containing mesenchymal stem cells can be prepared by flow cytometry or affinity chromatography. While a material used for obtaining this cell population is not limited, examples thereof include bone marrow and peripheral blood (including peripheral blood collected after G-CSF administration). Bone marrow may be collected from the spine, sternum, iliac bone, or the like. If the material used for cell preparation consists of aggregated spheres including mesenchymal stem cells, the material can be subjected to a mechanical treatment such as pipetting or an enzymatic treatment using trypsin, collagenase, etc., as necessary. Moreover, if the material is contaminated with red blood cells, the red blood cells are preferably hemolyzed in advance.

The cell population prepared as described above is used for sorting out CD271+CD90+ cells. In one exemplary method for sorting out CD271+CD90+ cells, antibodies are used. The antibodies are anti-CD271 and anti-CD90 antibodies that are capable of sorting out CD271+CD90+ cells. When flow cytometry is employed for sorting, an anti-CD271 antibody and anti-CD90 antibody labeled with different fluorescent dyes such as FITC, PE, or APC can be used in an appropriate combination to sort live cells in a short time. Other than flow cytometry, CD271+CD90+ cells can also be sorted by a method using magnetic beads or affinity chromatography.

Next, single-cell culturing (cloning) of the sorted LNGFR and Thy1-double-positive cells is performed to select a lot of rapidly proliferating cells, thereby obtaining high-purity human mesenchymal stem cells (REC: Rapidly Expanding Clones) excellent in proliferation potential, differentiation potential, and migration ability.

Mononuclear cells are prepared from human bone marrow or fat/placental chorion, and the bone marrow mononuclear cells are stained using anti-LNGFR antibody and anti-Thy1 antibody. Then, using flow cytometry (cell sorter), LNGFR-positive and/or Thy1-positive cells are cloned and sorted into a 96-well culture plate. Specifically, one cell per well is seeded. After 2 weeks of single-cell culturing, an image of the culture plate is photographed under a microscope to select wells that are confluent or semi-confluent and designate cells contained in these wells as RECs.

Herein, "rapidly proliferating" and "rapidly expanding" mean that, when one cell per well is seeded and cultured in a 96-well culture plate, the growth rate is such that the culture plate becomes confluent or semi-confluent within two weeks of culture (doubling time of 26±1 hour).

Confluent refers to a state where 90% or more of the surface of a culture container (surface of the culture) is covered by cultured cells. In addition, semi-confluent refers to a state where 70-90% of the surface of a culture container (surface of the culture) is covered by cultured cells. The size and type of the culture equipment used can be changed as appropriate depending on the growth rate of the cells. Cells that proliferate later on (moderately or slowly expanding cells), i.e., cells that are not semi-confluent or confluent after 2 weeks of single-cell culturing, are discarded. The RECs collected from each of the sorted wells are transferred to a culture flask for each well and further cultured to confluency (expansion culture). The cells from the expanded culture are then collected separately. RECs from one well are considered one lot (one clone in the examples described below) and will be used for the selecting described below.

Since the cells of the present invention are obtained by cloning and sorting where one cell is seeded per well, the genetic traits among all of the expanded cells are identical. Therefore, according to the present invention, the entire cell population may be referred to as a "clone" or each of the cells constituting the cell population may be referred to as a "clone."

According to the present invention, RECs used for selecting and quality evaluation can also be evaluated in advance using an REC marker (anti-Ror2). For example, after the aforementioned expanding culture, proliferating adherent cells are collected from all lots, and a portion (about 1 to 3×10³ cells) of each lot is stained with an anti-Ror2 monoclonal antibody for single staining. A technique of single staining using an anti-Ror2 monoclonal antibody is known (WO2016/17795). Briefly, the percentage of REC marker-positive cells in the collected cells is determined by flow cytometry analysis using an REC marker. The percentage can be determined by quantitating Ror2 mRNA expression using quantitative PCR, or can be determined manually by microscopy. Lots (cell populations) having a certain percentage of positive cells (e.g., 65%) are considered acceptable.

### 2. Characteristics of Cell Population

In the present invention, the characteristics of the cell population used as a reference standard for evaluating or determining the quality of cells of interest can be obtained from any of the following information (a) to (d). The cells of interest are not particularly limited, but mesenchymal stem cells, embryonic stem cells (ES cells), iPS cells, etc. are preferred.
(a) At least one piece of cell information selected from the group consisting of cell size, degree of spreading, contour complexity, pattern, and brightness of individual cells within the cell population, and image information thereof.

Information on cell size, degree of spreading, and contour complexity can be obtained, for example, by measuring or calculating indicators such as area, cell diameter, width, length, perimeter, arc length, combinations thereof, and the proportion of these features within the cell image (e.g., number of pixels).

Pattern and brightness can be obtained, for example, by using indicators such as the number of pixels showing a specific hue in the image.

(b) Information obtained by integrating the cell information of (a) across multiple cells within the cell population and statistically processing it.

The cell information of (a) is integrated across multiple cells within the cell population and subjected to statistical processing.

The method of statistical processing is not particularly limited.

Examples of the statistically processed information include mean value, standard deviation, and distribution shape.

(c) Combination information obtained by acquiring the information of (b) for multiple cell populations.

The information of (b) may be acquired for multiple cell populations and combined.

(d) Information of (a) to (c) acquired over time.

"Over time" means at any point after the preparation of the rapidly expanding mesenchymal stem cell population, after a predetermined time has elapsed, or continuously. Examples include information obtained 1 hour, 2 hours, 3 hours, 5 hours, or 10 hours later.

In the present invention, the quality of cells of interest used as an indicator for evaluation or determination includes cell proliferation rate, cell recovery number, degree of undifferentiation, degree of differentiation, amount of produced factors, and cell heterogeneity, but is not limited thereto.

Cell proliferation rate refers to the vigor of cell proliferation when the cells of interest or their progeny are cultured. For example, it can be evaluated or determined using indicators such as cell proliferation speed, number of passages until proliferation stops, and total number of cells obtained from one cell through proliferation. The cell proliferation rate is positively correlated with these indicators. When the proliferation rate is high, i.e., when these indicators are large, the quality can be evaluated or determined as high (high proliferation potential).

Cell recovery number refers to the number of viable cells obtained after culturing the cells of interest or their progeny for a certain period. When high proliferation potential is important, cells with a high recovery number can be evaluated or determined as "cells meeting the desired quality."

Degree of undifferentiation refers to the immaturity of the differentiation stage of the cells of interest in the process of functional differentiation of stem cells, or the diversity of cell types into which the cells of interest can differentiate. When it is important to prepare and store large quantities of stem cells in an undifferentiated state, cells with a high degree of undifferentiation can be evaluated or determined as "cells meeting the desired quality."

Degree of differentiation refers to the extent or speed at which the cells of interest normally differentiate into the target cell type, tissue, or organ. When it is important to differentiate stem cells to the desired state, cells with a high degree of differentiation can be evaluated or determined as "cells meeting the desired quality."

Amount of produced factors refers to the amount (e.g., concentration) or number of types of specific cytokines produced by the cells of interest. Based on the amount of produced factors, it is possible to evaluate or determine whether the cells of interest possess the desired functions (e.g., proliferation potential, migration ability), the degree of such functions, and the degree of aging of the cells of interest. For example, when the amount of cell growth factors produced by the cells of interest is high, proliferation potential is high; when the amount of migration-promoting factors is high, migration ability is high. In either case, the degree of conformity to the desired cell quality can be evaluated or determined.

Cell heterogeneity refers to the lack of homogeneity in the phenotype of the cells of interest (including morphological characteristics, migration ability, responsiveness, and other cellular states). It indicates the possibility that a cell population meeting the desired quality cannot be produced with high purity (risk of manufacturing failure). Therefore, low heterogeneity indicates that process control parameters are being properly managed for producing cells with the desired quality; high heterogeneity indicates a risk that culture control is insufficient. When heterogeneity is sufficiently low, it can be evaluated or determined that the cell manufacturing capability is high (process control is adequate), or the cell manufacturing success rate is high (low culture risk, high economic efficiency and productivity).

### 3. Information Providing Method, Quality Evaluation Method, and Systems and Programs Used in These Methods

### (1) Information Providing Method and Quality Evaluation Method

The present invention provides a method for providing information for evaluating the quality of a stem cell population of interest, and a method for evaluating the quality of a stem cell population of interest, in which the cell reference standard of the present invention is used.

The information providing method of the present invention comprises the steps of:
(a) characterizing individual cells of a rapidly expanding mesenchymal stem cell population by using, as an indicator, the positivity or negativity of at least one marker selected from LNGFR (CD271) and Thy-1 (CD90), and acquiring image data (reference image information) of the individual cells, or acquiring parameter information (reference parameter information) representing characteristics of the cell population from the reference image information;
(b) acquiring image data (image information of interest) of individual cells of a stem cell population of interest provided by a user, or acquiring parameter information (parameter information of interest) representing characteristics of the stem cell population of interest from the image information of interest;
(c) comparing the image information of interest or parameter information of interest with the reference image information or reference parameter information; and
(d) providing to the user all or part of the information obtained by the comparison for evaluating the quality of the stem cell population of interest.

The present invention also provides a method for evaluating the quality of cells of interest, the method comprising the step of:
(d) evaluating the quality of stem cells of interest by using, as an indicator, all or part of the information obtained by the comparison in step (c).

These methods may use systems and programs including units for implementing steps (a) to (d). The system of the present invention is described below.

### (2) Information Processing Device and Program

An information processing device and program for quality evaluation according to one embodiment of the present invention will be described with reference to the drawings.

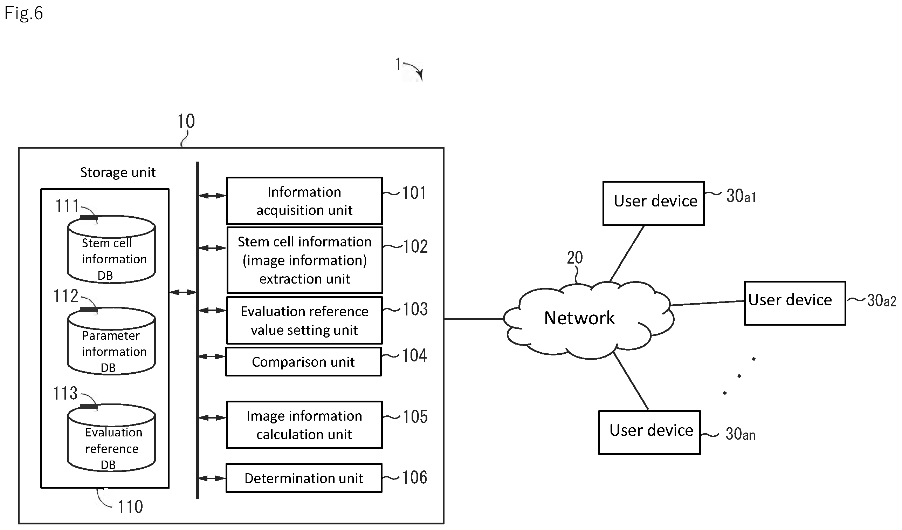

Fig. 6 is a schematic configuration diagram of a system 1 for evaluating the quality of a stem cell population of interest, including an information processing device 10 according to this embodiment. In the following description, institutions handling cells of interest and institutions or individuals wishing to obtain stem cell information (inspection companies, hospitals, universities, etc.) are referred to as "users."

The information processing device 10 is a server-type computer equipped with a CPU, ROM, RAM, input/output interfaces, and the like. The information processing device 10 is communicably connected via a network 20 such as the Internet or LAN to user devices 30ₐ₁, 30ₐ₂, ... 30ₐₙ (collectively referred to as "user device 30"). The number of user devices 30 may be one or more.

The information processing device 10 characterizes individual cells of a rapidly expanding mesenchymal stem cell population serving as the cell reference standard by using, as an indicator, the positivity or negativity of at least one marker selected from LNGFR (CD271) and Thy-1 (CD90), and acquires image data (reference image information) of the individual cells, or acquires parameter information (reference parameter information) representing characteristics of the cell population from the reference image information.

Next, the information processing device 10 compares items of stem cell information (including image information) of a user handling cells of interest ("target user") with corresponding items of stem cell information of the REC reference standard stored in the database, and calculates and acquires parameter information based on the comparison results.

Then, the information processing device 10 compares the calculated parameter information and evaluates and determines the quality of the stem cell population of interest of the target user. The information processing device 10 then transmits the user's stem cell information and the evaluation and determination results to the user device 30.

The user device 30 is a computer equipped with information input and display units, or a smartphone or tablet device, and is communicably connected to the information processing device 10 via the network 20. The user device 30 displays various types of information received from the information processing device 10 on a display through a browser or application, and provides an interface for inputting information to be sent to the information processing device 10.

The information processing device 10 includes functional units realized by cooperation between programs stored in ROM or placed in RAM and the CPU, including: an information acquisition unit 101, a stem cell information extraction unit 102, an evaluation reference value setting unit 103, a comparison unit 104, an image information calculation unit 105, and a determination unit 106. The information processing device 10 also includes a storage unit 110 provided in ROM (or RAM), and the storage unit 110 includes a stem cell information database (DB) 111, a parameter information database (DB) 112, and an evaluation reference database (DB) 113.

The information acquisition unit 101 acquires various types of information such as stem cell image information from the user device 30 via the network 20 and stores it in the storage unit 110. Information received from the user device 30 includes stem cell information obtained by the user and user identification information (user name, stem-cells-of-interest information such as source and provider, or user ID, etc.).

When the information acquisition unit 101 receives stem-cells-of-interest data from a user, it stores the data in the stem cell information DB 111 in association with the user identification information. The stem cell information DB 111 stores stem cell data collected from multiple users in this manner. When the information acquisition unit 101 receives stem cell information from a user, it acquires parameter information representing the characteristics of the stem cell population of interest from the stem cell information and stores it in the parameter information DB 112. The evaluation reference DB 113 stores information obtained by statistical analysis processing performed by the comparison unit 104 to extract characteristics. This information is stored in association with information identifying the user population sample subjected to the statistical analysis.

Statistically processed information includes any of mean value, standard deviation, and distribution shape, or combinations thereof. Details of these types of information are as described above.

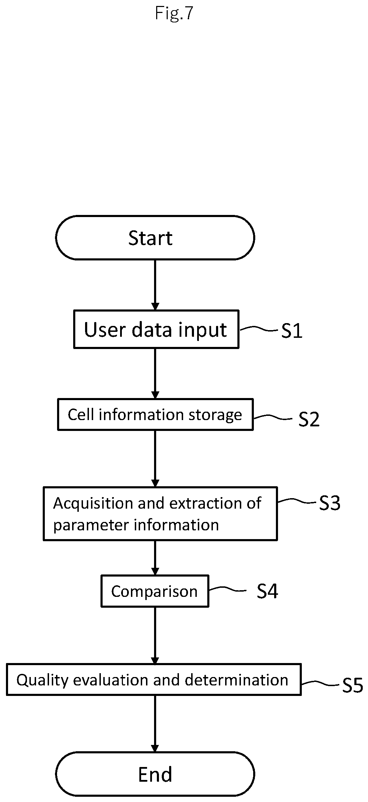

Fig. 7 is a processing flowchart of the information processing device 10 according to this embodiment.

First, the information acquisition unit 101 of the information processing device 10 requests input of basic information and stem-cells-of-interest information such as cell images from the user and acquires this information (S1), and stores it in the stem cell information DB 111 (S2).

The stem cell information extraction unit 102 extracts from the information stored in the stem cell information DB 111 at least one indicator selected from the group consisting of cell proliferation rate, cell recovery number, degree of undifferentiation, amount of produced factors, and cell heterogeneity, which serve as indicators of quality, and stores it in the parameter information DB 112 (S3). The evaluation reference value setting unit 103 sets determination reference values for each quality evaluation item based on the information stored in the parameter information DB 112 and stores them in the evaluation reference DB 113.

The comparison unit 104 extracts stem cell population information (reference parameter information) of the reference standard and stem cell information (parameter information of interest) of the user from the stem cell information DB 111 and the parameter information DB 112, and compares the stem cell information between the reference parameter information and the parameter information of interest (S4).

The image information calculation unit 105 calculates numerical values for quality evaluation of the stem cells of interest based on the comparison results between the reference parameter information and the parameter information of interest, and acquires information for quality evaluation and determination (S5). The determination unit 106 determines the quality of the stem cells of interest of the target user. The information processing device 10 refers to the data stored in the evaluation reference DB and determines whether the quality of the cells is good or poor based on the reference values, or displays evaluation scores. The display results are shown to the user on the user device via a display unit (not shown).

The program of the present invention can be stored in a computer-readable recording medium or a storage unit connectable to a computer. A computer-based recording medium or storage unit containing the program of the present invention is also included in the present invention. Examples of recording media or storage units include magnetic media (flexible disks, hard disks), optical media (CDs, DVDs), magneto-optical media, and flash memory, but are not limited thereto.

### EXAMPLES

The present invention will be described in further detail below with reference to Examples. However, the scope of the present invention is not limited to these Examples.

### [EXAMPLE 1]

Mesenchymal stem cells (MSCs) are the most extensively studied stem cells for applications in cell therapy [1-3]. It is known that various cell types exist within MSCs [4-7], because conventional MSC manufacturing simply collects the adherent cell fraction from a mixture of cell suspensions [8, 9]. LNGFR (CD271) and THY-1 (CD90) double-positive cells (LT cells) are a specific MSC subpopulation in bone marrow, and they exhibit unique characteristics that contribute to the advancement of MSC-based cell therapy, including: (1) high proliferation potential, (2) multipotent differentiation potential (adipogenic, osteogenic, and chondrogenic differentiation), (3) low expression of the senescence marker SA-β-gal, and (4) small and homogeneous morphology that enables avoidance of entrapment in pulmonary capillaries after intravenous administration in mouse models [10].

Relatively rapidly proliferating clones were observed among single-cell-sorted LT cells and were named "Rapidly Expanding Clones (REC)." Because the proliferative potency of REC greatly contributes to establishing cell banks with reduced heterogeneity, REC is currently under clinical trials for hypophosphatasia and lumbar spinal stenosis [11].

Since MSCs are used in many studies for clinical applications [3, 12-14], expectations for the advancement of MSC-based cell therapeutics are increasing. Therefore, currently, there is a strong demand for technologies that can support MSC manufacturing [15, 16]. REC offers significant advantages because its characteristics reduce two major risks in current MSC manufacturing processes and increase the feasibility of constructing efficient and robust cell manufacturing processes.

### (1) Difficulty in controlling donor-to-donor quality variability

MSCs exhibit substantial variability among donors [17-19], and it is practically impossible to sufficiently investigate donor cell variability before developing a robust manufacturing process [20-22]. Thus, handling various unknown donor cells is a highly risky task in MSC manufacturing and is a major cause of unexpected and difficult-to-resolve errors.

Establishing an allogeneic cell bank is a practical approach for controlling the quality of starting cells, enabling efficient MSC manufacturing. REC can be highly beneficial in the process of establishing allogeneic cell banks. Since REC can be mass-produced by cell sorting, selecting REC is more feasible and efficient than the difficulty of performing large-scale donor screening to obtain an ideal cell bank. Furthermore, the proliferation potential of REC significantly increases the success rate of establishing cell banks while maintaining banked cells during the early culture period. Therefore, manufacturing using REC as the starting cell enables more feasible process development and stable quality control.

### (2) Quality deterioration during cell proliferation culture

MSCs lose proliferation potential after several passages, and other important quality attributes also decline during expansion culture [23-31]. However, achieving a certain number of cells is an essential quality requirement in the manufacture of MSC-based therapeutic products. From a therapeutic perspective, typical MSC-based treatment protocols require more than one billion cells per treatment to ensure efficacy [32]. From a manufacturing perspective, most quality tests are invasive and consume cells; therefore, the final cell bank must reach a large cell number to allow comprehensive testing across many quality criteria [33].

However, such cell manufacturing processes are attempts without guaranteed success because the possibility of quality deterioration cannot be excluded. In practice, failure to establish a cell bank can only be discovered *after* all work has been completed, and it is difficult to prevent such failures in advance. In this context, the high and sustained proliferation potential of REC, which enables more than 10 serial passages, greatly minimizes the risk of failure in establishing a cell bank and allows maximum culture efficiency to establish abundant stock at early passages.

Although REC offers the advantages of enabling low-risk and efficient manufacturing, there is a dilemma in establishing cell banks for REC. REC can be effectively produced by cell sorting even from a small cell source. Therefore, variation among starting clones can be greater than that of allogeneic cell banks. However, expanding many REC clones to the final cell bank stage is costly, and final quality testing required to guarantee the established cell bank may incur additional cost and labor.

Variation among starting REC clones creates the expectation that "better REC" can be selected during cell bank establishment, but such expectations increase the number of cell banks that must be established, requiring additional cost and labor. In practice, REC is screened from LT cells based on early proliferation speed from single clones in 96-well plates, and all candidates are further expanded to form multiple "candidate cell banks," from which "better REC cell banks" with higher quality are selected.

Although REC allows greedy selection for higher-quality products (i.e., final products with lower risk and higher potency), this may increase the cost of the entire process. Therefore, it is necessary to identify such candidate REC as early as possible to minimize excessive work. However, using conventional cell evaluation methods to examine early-stage cells in cell bank establishment, especially single-clone-derived REC, is extremely difficult because most methods are invasive and waste valuable cell sources.

In this study, a morphology-based non-invasive potency prediction method was developed to select "better REC" at the early stage of cell bank establishment, where cell numbers are extremely limited. Based on the authors' previous findings regarding morphology-based early prediction of MSC quality deterioration [34-37], the authors attempted to predict "further serial passage potency" using only early morphological information to enhance the selection of better REC capable of forming better REC cell banks (Fig. 1).

Furthermore, such potency prediction is expected to assist in the cell bank establishment process, which balances the critical dilemma for processing cell banks: "bank size" and "bank cell potency." If a cell bank is expanded extensively, manufacturing efficiency can increase, but this increases the risk of losing the proliferation potential of banked cells. Therefore, if future serial passage potency can be predicted in advance, it would be possible to determine a low-risk timing to achieve the largest possible cell bank with high potency.

For training data to develop the prediction model, the inventors established 15 REC clones from bone marrow and experimentally confirmed the number of serial passages until the passage limit (defined as "serial passage potency"). In this Example, morphological descriptors at passage 1 (P1) stage were used to develop a machine learning model for quantitatively predicting such potency. During the development of the prediction model, detailed analysis of data utilization effects was conducted to robustly obtain high-performance prediction models. Thus, the inventors' data demonstrated the most effective time-course data usage and minimum number of images required to implement the prediction model in a practical manner. The inventors' concept of cell morphology-based prediction of future REC potency suggests that an image-based, data-driven cell bank construction process in MSC manufacturing may achieve both efficiency and robustness.

### Materials and Methods

### <Cells and Culture>

To obtain REC, bone marrow mononuclear cells were prepared from bone marrow aspirates collected from healthy donors (AllCells, Alameda, USA) using density gradient centrifugation with Ficoll (GE Healthcare, Chicago, USA). Bone marrow mononuclear cells were stained at 37°C for 1 hour with anti-human rabbit anti-CD90 IgG (BD Biosciences, Cat#559869, Franklin Lakes, USA) and anti-human mouse anti-CD271 IgG (Thermo Fisher).

Single-cell sorting of CD90- and CD271-double-positive cells was performed using a cell sorter (JSAN, BayBioscience, Kobe, Japan). LT cells were further cultured as single clones (passage number = P0) in 96-well plates (Thermo Fisher Scientific, Waltham, USA) using maintenance medium consisting of low-glucose Dulbecco's Modified Eagle Medium [DMEM] (Wako, Osaka), supplemented with 20% fetal bovine serum [FBS] (Cytiva HyClone, Marlborough, USA), 20 ng/ml basic fibroblast growth factor [bFGF] (KAKEN PHARMACEUTICAL, Tokyo, Japan), 0.01 M 4-(2-hydroxyethyl)-1-piperazineethanesulfonic acid [HEPES] (Thermo Fisher Scientific, Waltham, USA), and 1% penicillin/streptomycin (Meiji Seika Pharma, Tokyo, Japan). After 2 weeks, clones were grown to sub-confluence in the 96-well plates, collected, and subcultured in a single well of a 6-well plate (Thermo Fisher Scientific) (passage number = P1), where they were prepared for image acquisition.

Image acquisition for further morphological analysis was performed at this stage. Next, when the cells in the 6-well plates (Thermo Fisher Scientific) reached sub-confluence, they were collected and subcultured in T75 flasks (Thermo Fisher Scientific) (passage number = P2). Cells were collected using TrypLE Select (Thermo Fisher Scientific) after incubation at 37°C for 3-5 minutes.

P2 cells in T75 flasks that had reached sub-confluence were cryopreserved at 1.0 × 10⁶ cells/ml using CP-1 (KYOKUTO PHARMACEUTICAL, Tokyo, Japan) and 25% human serum albumin (CSL Behring, Tokyo, Japan), and designated as the "primary cell bank (P3)." To preserve cells for serial passage tests, in vitro and in vivo assays for multiple potencies were excluded from the primary cell bank.

For morphological comparison, conventionally processed bulk MSCs (BMC; Lot 0000394413, 0000411107, 0000413042, 0000422610, 0000423370, 0000429365, 0000446319, 0000451491, 0000458207) purchased from Lonza Japan, Ltd. (Tokyo, Japan) were cultured to P3 in MSCGM (Lonza Japan, Ltd.) supplemented with BulletKit (Lonza Japan, Ltd.). All cultures were maintained at 37°C with 5% CO₂ and medium was changed every 3 days.

### <Serial Passage Potency Test>

A vial of the primary cell bank (P3) was thawed, and cells were seeded at 2.0 × 10⁵ cells/dish in 100-mm dishes (Thermo Fisher Scientific), designated as P3 data. Cells were collected from the 100-mm dishes using TrypLE Select (Thermo Fisher Scientific), and when they reached sub-confluence, they were subcultured into new 100-mm dishes at the same seeding density. This passaging process was repeated until cell proliferation ceased. The number of passages was counted, and the final passage number was designated the "limit number of serial passages," which was defined as "serial passage potency" (Fig. 1). At each passage, the total number of collected cells was counted using a Cellometer Auto T4 (Nexcelom Bioscience, Lawrence, USA) and recorded as the total number of recovered cells.

### <Image Acquisition and Processing>

For P1 REC, phase-contrast microscopic images were acquired every 6 hours using an automated imaging system, BioStation CT (Nikon Corporation, Japan), at 4× magnification (64 tiles/well, covering 16 mm², 1000 pixels/image) in 6-well plates (Thermo Fisher Scientific). Each clone lot was imaged in one well. At this stage, the number of cells was extremely limited.

For bulk MSCs, MSCs were seeded at 2000 cells/cm² in 6-well plates (Corning Incorporated, NY, USA) (n = 3 wells per lot). Time points were designated as time point 1 (6 hours after seeding) through time point 20 (120 hours after seeding). Time point 15 was selected as the final time point for measuring morphology and proliferation rate because some lots reached sub-confluence or higher, making it difficult to accurately identify individual cells in the images.

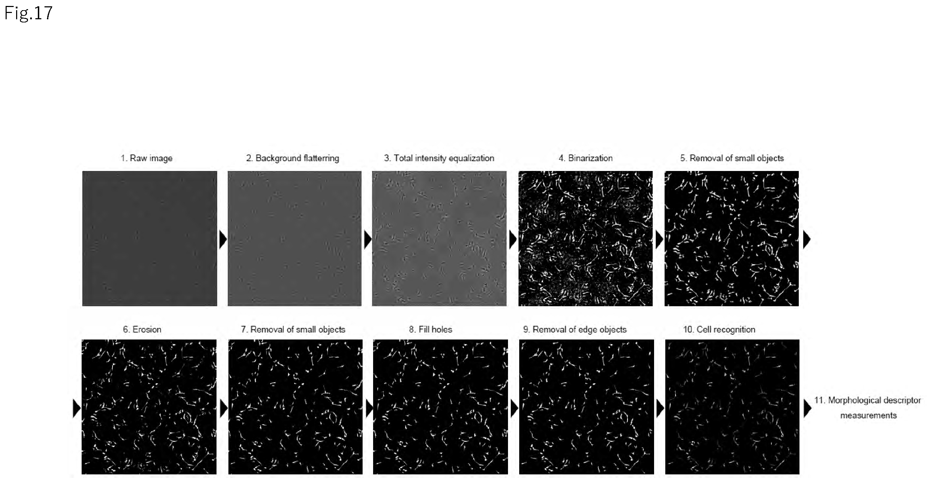

Raw images were processed to obtain summaries of morphological profiles of the cell populations by measuring individual cell morphology (from a minimum of 300 cells to a maximum of 100,000 cells per well, covered by 64 images). Image processing was performed using original code written in Python version 3.7.3, with NumPy version 1.20.0 and OpenCV version 4.4.0. Eight processing steps were designed: 1) background adjustment, 2) texture enhancement, 3) binarization, 4) removal of small objects, 5) erosion, 6) removal of small objects, 7) fill holes, and 8) removal of frame-touching objects (Fig. 17). After image processing, 12 basic morphological descriptors (Table 1) were measured for each cell region in each image.

**[Table 1]**

| Supplementary Table 1. Basic morphological parameters measured per single cells. | | | |
|---|---|---|---|
| Number | Parameter type | Name | |
| 1 | Shape descriptors | Area | Total pixels in the recognized cell region. |
| 2 | | Compactness | (Perimeter)^2/Area |
| 3 | | Inner radius | Radius of inscribed circle from the centroid of cell area. |
| 4 | | Length width ratio | Length/Width |
| 5 | | Perimeter | The arc length of recognized cell region. |
| 6 | | Shape factor | 4π(Area)/(Perimeter)^ 2 |
| 7 | | Correlation | Gray-Level Co-occurrence Matrix (GLCM) of cell region. |
| | | | GLCM = M |
| | | | Correlation |
| | | | $= {\sum }_{i , j} \frac{\left(i-{μ}_{x}\right) \left(j-{μ}_{y}\right) {M}_{i ,}}{{σ}_{x} {σ}_{y}}$ |
| | | | ${μ}_{x} = {\sum }_{i , j} {jM}_{i , j}$ |
| | | | ${μ}_{y} = {\sum }_{i , j} {iM}_{i , j}$ |
| 8 | Texture descriptors | Energy | Gray-Level Co-occurrence Matrix (GLCM) of cell region. |
| | | | GLCM = M |
| | | | $Energy = {\sum }_{i , j} {M}_{i , j}^{2}$ |
| 9 | | Entropy | Gray-Level Co-occurrence Matrix (GLCM) of cell region. |
| | | | GLCM = M |
| | | | $\begin{matrix}Entropy \\ = - {\sum }_{i , j} {M}_{ij} {logM}_{i , j}\end{matrix}$ |
| 10 | | Inertia | Gray-Level Co-occurrence Matrix (GLCM) of cell region. |

Summaries of cell populations were described by calculating the "mean" and "standard deviation (SD)" of all single-cell-based morphological descriptor data. These summaries represented approximately 1 × 10⁴ to 1 × 10⁵ cells from a single well covered by 64 images.

The inventors accumulated summaries of these morphological descriptors over time (6-90 hours) and designated them as the "morphological profile" of the sample. A complete morphological profile for each condition (= one well) contained 360 parameters (= 12 descriptors × (mean and SD) × 15 time points). All data processing was performed using R version 4.0.2.

### <Visualization of Morphological Profiles>

Using single-cell data at each time point, Gaussian kernel density estimation was performed to visualize the cell population using a single morphological descriptor. The distribution was estimated using the kernel function in R. For distribution estimation, raw data were transformed using log10, and "area" was described. Measurement data of single cells with an area >200 pixels were collected, enabling detailed discussion of "area" between REC and conventionally processed bulk MSCs.

Many objects smaller than 200 pixels were found among "round cells" during proliferation; therefore, because the characteristics of enlarging cells decrease in such data, it was considered difficult to discuss size differences. Morphological profiles of all lots were analyzed using principal component analysis (PCA), and the relative similarity of clones was visualized using multiple descriptors.

In the PCA comparing morphological profiles of clones, dots were colored according to clone labels (15 colors) and serial passage limit numbers (7-level gradient: 7-13). All data with the same time-window size, including those using different numbers of FOVs, were integrated and used to set all principal components covering the full data diversity, visualize data utilization effects, and vary the time windows and FOV numbers.

Next, data obtained using different numbers of FOVs were individually plotted on fixed PCA axes. In the comparative PCA for data utilization effects, one plot represented "one clone." By resampling all single-cell measurement data for each data size (varying combinations of different time-window sizes and FOV numbers) using bootstrap (50 iterations with replacement), the explanatory power of different data usages was visualized, and new means and SDs were recalculated from each resampled dataset. Using PCA and these resampled datasets, a total of 50 plots per clone were generated. Student's t-test was used to examine differences between morphological descriptors and population distributions of morphological descriptors. All data processing was performed using R (version 4.0.2).

### <Constructing a Model for Predicting Serial Passage Potency>

Time-course morphological profiles were used as explanatory parameters, and experimentally determined serial passage limit numbers were used as objective parameters for the machine learning dataset. Two types of machine learning models were evaluated: LASSO regression (a linear regression model) and random forest (RF) (a nonlinear machine learning model). In LASSO, parameter selection was performed using the mean decrease Gini index. Model performance was validated using leave-one-out cross-validation and compared using the root mean square error (RMSE).

The data usage effect in morphological information was examined using exhaustive combinations of two parameters: time-window length (range: 6-90 hours) and number of FOVs (range: 1-64). To vary the time-window length, the window was shortened by a total of 6-90 hours from the final time point (90 hours) at each time point (6 hours). Thus, the maximum number of total morphological descriptors was 360 parameters, and the minimum was 24 parameters. Images were randomly selected from the 64 images to vary the number of FOVs. Bootstrap resampling (50 iterations) of FOVs from the 64 images was introduced, increasing the dataset size from 15 to 750 samples, and the effect of data variation was evaluated for selected data-size conditions (6 hours, 6-30 hours, 6-60 hours vs. FOV 15, 40, and 60). All data processing and machine learning were performed using R version 4.0.2.

### Results

### <Obtaining and Characterizing REC for Training Data>

To develop a machine learning model that predicts "serial passage potency" from early-stage cell morphology, we began work on obtaining 15 REC clones from bone marrow mononuclear cells (BMMCs) derived from a single donor (scheme in Fig. 1). The 15 clones were obtained from the same donor sample and sorted out according to the basic criteria for REC: (1) being LNGFR- and THY-1-double-positive at the time of cell sorting, and (2) reaching sub-confluence within two weeks after single-clone sorting into a 96-well plate (P0 stage).

Next, clones expanded in 6-well plates and subsequently in T-75 flasks were cryopreserved as a primary cell bank (P3), which stores early-passage cells.

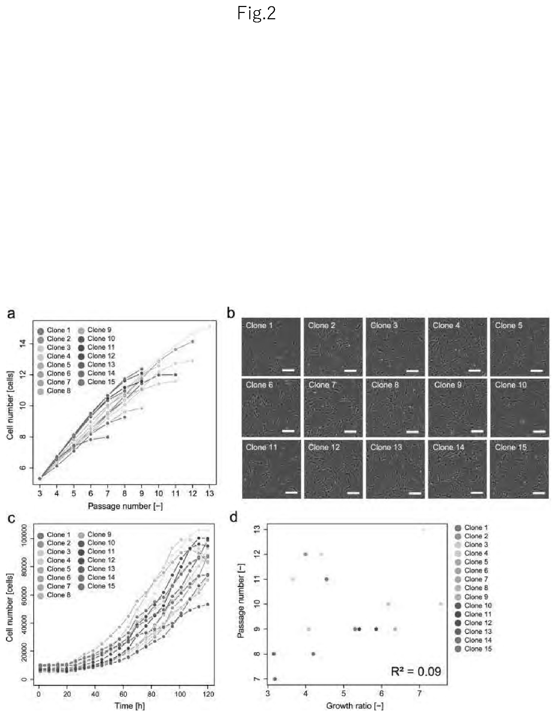

To further select "better REC," the serial passage potency of candidate clones at P2 was evaluated. The ability to undergo multiple repeated passages can be considered an ideal criterion for selecting "higher-quality cell banks" for further use. Results of the serial passage potency test showed that REC retained high proliferation potential, recording an average of 9.8 serial passages (Fig. 2a). Such performance can be considered superior compared with commercially available MSCs processed using conventional methods.

Next, morphological characteristics of the clones were evaluated (Fig. 2b). At the P1 stage in 6-well plates (a very early stage), morphology contains important signatures for evaluating the cells. However, manual morphological observation (without quantification) makes it difficult to distinguish differences among REC.

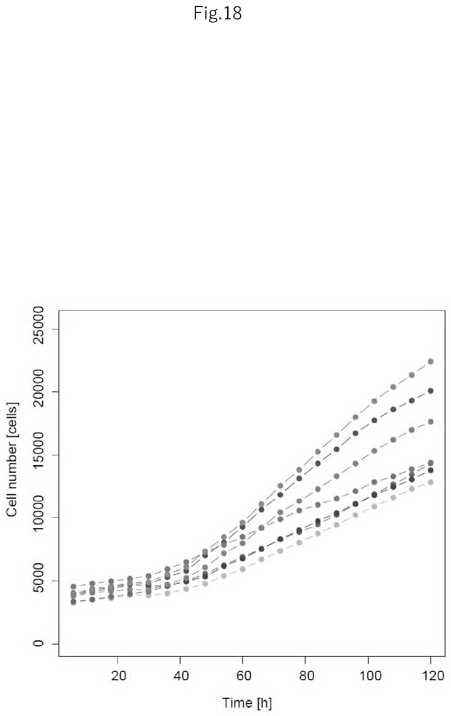

The proliferation profiles of REC at the P1 stage were analyzed using time-course images (Fig. 2c). Of the 15 clones, 12 exhibited a proliferation rate exceeding four-fold. This proliferation rate was significantly higher than that of multiple conventionally processed bulk MSCs in the inventors' study (Fig. 18). The clone with the lowest serial passage potency (clone 13) showed a low proliferation rate, whereas the clone with the highest serial passage potency (clone 4) showed a high proliferation rate. However, the coefficient of determination between "proliferation rate" and "limit number of passages" was low (R² = 0.09) (Fig. 2d). Thus, these data indicate that proliferation rate measurements at the P1 stage cannot predict future serial passage potency.

### <Morphological Characteristics of REC>

Following known analytical methods [33-36], the inventors attempted quantitative characterization of REC (P1 time point) using image-based morphological analysis. Compared with conventionally processed bulk MSCs (cMSC, 9 lots), REC were more uniform and remained smaller even after adhesion (Figs. 3a, 3b). Both REC and cMSC started from cell populations of similar size at the very early adhesion stage (6 hours, t-test p < 0.22) (median area = 358 µm² and 335 µm², respectively). The median size of REC remained small (363 µm²), whereas bulk cMSC enlarged by 30 hours after culture (median area = 473 µm², t-test p < 0.000001). Furthermore, in this series of time-course images, more proliferating cells (appearing white and round under phase-contrast microscopy) were observed in REC (Fig. 3b).

Visualization of population changes in REC at P1 revealed that the adherent cell populations of clones with reduced serial passage potency remained larger in cell area (Fig. 3c). Thus, high-performance cells form a uniform cell-size population; however, such size-data analysis is only suggestive, and the coefficient of determination between "SD of area" and "limit number of passages" was low (Fig. 3d). Analysis using a single morphological descriptor was not sufficiently effective for quantitative prediction.

Clones were profiled using multiple morphological descriptors derived from 24 descriptors obtained from basic descriptors (Table 1) [33-36]. Morphological similarity among REC was visualized using principal component analysis (PCA) (Fig. 3e shows clone numbers; Fig. 3f shows serial passage potency of each clone).

These results indicate that specific clusters of clones with similar morphological profiles exist, and these clusters slightly separate low-serial-passage-potency clones from high-serial-passage-potency clones. In practice, clones with low serial passage potency cluster at lower positions on the PC2 axis, whereas clones with high serial passage potency cluster at higher positions on the PC2 axis and near the center of the PC1 axis. The descriptors contributing to each axis of the PCA map, particularly the most explanatory PC2 axis, can be interpreted as follows (Table 2).

**[Table 2]**

| Supplementary Table 2. Morphological descriptors highly contributing to PCA. | | | |
|---|---|---|---|
| PC1 | | PC2 | |
| Parameters | Factor loadings | Parameters | Factor loadings |
| 84h_mean_energy | 0.08 | 24h_mean_length_width_ratio | 0.11 |
| 78h_mean_energy | 0.08 | 30h_mean_length_width_ratio | 0.10 |
| 72h_mean_energy | 0.08 | 18h_mean_perimeter | -0.10 |
| 54h_mean_length | -0.08 | 12h_mean_length | -0.10 |
| 60h_mean_perimeter | -0.08 | 18h_sd_width | -0.10 |
| 78h_mean_intensity_sd | -0.08 | 24h_sd_perimeter | -0.10 |
| 60h_mean_length | -0.08 | 24h_sd_length | -0.10 |
| 54h_mean_area | -0.08 | 18h_sd_compactness | -0.11 |
| 72h_mean_intensity_sd | -0.08 | 18h_mean_length | -0.11 |
| 54h_mean_perimeter | -0.08 | 24h_mean_length | -0.11 |

When cells become more uniform and spindle-shaped during 24-30 hours of proliferation, the clone possesses higher serial passage potency (i.e., can undergo long-term serial passage), whereas disruption of cellular morphological uniformity leads to reduced serial passage potency. Such unsupervised model analysis suggests that combinations of multiple descriptors provide better explanations for morphologically characterizing performance.

### <Morphology-Based Machine Learning for Predicting Serial Passage Potency>

Next, to enable quantitative prediction of the "serial passage potency" of REC based solely on morphological information, the construction of a machine learning model using morphological information was examined (Fig. 1). Using morphological profiles (24 descriptors × 15 time points), the limit number of serial passages was predicted. In constructing this prediction model, we attempted to understand which parts of the morphological information, or to what extent, effectively contribute to the development of the prediction model.

Therefore, the data usage effect of morphological data was examined by varying two parameters: time window effect and number-of-FOV (field of view) effect. These parameters were examined because, in the authors' previous studies attempting to predict MSC proliferation rate and osteogenic differentiation speed from morphological descriptors, detailed examination of these parameters was found to effectively reduce the effort required for image data collection [33-36]. Shortening the time window and minimizing the number of FOVs not only saves time and labor for image acquisition but also improves prediction efficiency.

By thoroughly examining both the "time window effect" and the "FOV number effect" using LASSO regression, it was found that high-performance prediction models could be obtained using morphological information from P1 cells with several combinations of parameters (shown as green heat-map regions with RMSE < 1.0 in Fig. 4a). These data suggest that prediction performance can be maintained even when the time window and number of FOVs are reduced. Furthermore, it was inferred that the effect of "number of FOVs" is more important than the effect of "time window," because when more than 15 FOVs are collected, the time window can be shortened without reducing performance.

Scatter plots were generated to further understand the performance of the prediction model for serial passage potency for each clone (Fig. 4b). These data clearly demonstrate that the inventors' prediction model predicts the "quantitative value of the limit number of serial passages" (Figs. 4a, 4b). Many high-performance prediction models were developed using training datasets of various sizes. However, because LASSO is an algorithm that generates an optimal combination of descriptors for each dataset, all model structures differ randomly and lack a common structure, raising the possibility that models with similar performance but completely different structures may be generated.

When model structures differ across data conditions, such modeling results are not robust and therefore not practical. Thus, correlations among all model structures were compared (Fig. 4c), and it was confirmed that high-performance models robustly share similar model structures. These results suggest that the relationship between combinations of morphological descriptors and serial passage potency can be modeled using a specific universal combination of morphological descriptors.

Comparisons of highly contributing descriptors shared among different models, such as "Correlation_SD (18h)," "Correlation_SD (6h)," and "Energy_SD (18h)," showed positive contributions to predicting clones with high serial passage potency. Conversely, "Correlation_mean (6h)," "Length_SD (18h)," and "Compactness_SD (18h)" contributed negatively to predicting clones with low serial passage potency (Table 3).

**[Table 3]**

| Supplementary Table 3. Morphological descriptors used in LASSO models. | | | | | | | |
|---|---|---|---|---|---|---|---|
| Model using 15FOVs_6-18h | | Model using 40FOVs_6-18h | | Model using 60FOVs_6-18h | | Model using 60FOVs_6-90h | |
| Parameter | Coefficient | Parameter | Coefficient | Parameter | Coefficient | Parameter | Coefficient |
| 18h_mean_perimeter | 1.781 | 18h_mean_energy | 1.191 | 18h_mean_area | 0.753 | 36h_mean_intensity_total | 0.226 |
| 6h_mean_area | 1.391 | 18h_mean_lntensity_sd | 0.865 | 6h_sd_correlation* | 0.582 | 18h_sd_correlation | 0.075 |
| 18h_mean_intensity_sd | 1.104 | 18h_sd_perimeter | 0.845 | 18h_sd_energy** | 0.559 | 6h_sd_correlation* | 0.069 |
| 18h_mean_energy | 1.092 | 18h_mean_area | 0.668 | 18h_mean_energy | 0.436 | 12h_sd_correlation | 0.057 |
| 6h_mean_lenglh | 0.904 | 6h_sd_correlation* | 0.629 | 18h_sd_width | 0.391 | 36h_mean_width | 0.046 |
| 6h_sd_correlation* | 0.662 | 6h_mean_area | 0.447 | 18h_mean_intensity_sd | 0.345 | 48h_mean_intensity_total | 0.041 |
| 18h_sd_perimeter | 0.587 | 18h_sd_energy** | 0.364 | 12h_mean_area | 0.261 | 24h_mean_width | 0.040 |
| 6h_mean_width | 0.537 | 6h_sd_energy | 0.325 | 6h_mean_area | 0.156 | 18h_sd_energy** | 0.039 |
| 18h_mean_area | 0.522 | 12h_mean_width | 0.319 | 6h_mean_compactness | 0.136 | 30h_mean_width | 0.036 |
| 6h_sd_intensity_total | 0.485 | 12h_sd_perimeter | 0.295 | 6h_mean_lenglh | 0.127 | 78h_sd iniensity_mean | 0.028 |
| 12h_mean_length_width_ratio | -0.539 | 16h_mean_homogeneity | -0.437 | 18h_mean_homogeneity | -0.220 | 6h_mean_correlation** | -0.083 |
| 6h_mean_energy | -0.565 | 6h_mean_perimeter | -0.448 | 6h_sd_perimeler | -0.231 | 54h_sd_length_width_ralio | -0.097 |
| 18h_mean_compactness* | -0.642 | 6h_mean_correlation** | -0.479 | 18h_mean_intensity_tolal | -0.269 | 42h_mean_length_width_ratio | -0.109 |
| 18h_sd_lenglh | -0.673 | 6h_mean_length_width_ratio | -0.493 | 12h_mean_correlation | -0.287 | 90h_mean_area | -0.110 |
| 12h_mean_homogeneity | -0.683 | 18h_sd_intensity_sd | -0.498 | 6h_mean_length_width_ratio | -0.412 | 18h_sd_length* | -0.112 |
| 12h_mean_perimeter | -0.784 | 12h_mean_perimeler | -0.519 | 12h_sd_length | -0.431 | 48h_mean_length_width_ratio | -0.132 |
| 18h_mean_length_width_ratio | -0.865 | 12h_mean_length_width_ratio | -0.577 | 6h_mean_correlation** | -0.484 | 18h_sd_compactness* | -0.242 |
| 6h_mean_perimeter | -1.135 | 18h_sd_compactness* | -0.688 | 18h_mean_length_width_ratio | -0.511 | 24h_sd_compactness | -0.265 |
| 6h_mean_intensity_total | -1.594 | 18h_mean_length_width_ratio | -0.825 | 18h_sd_cornpactness^{a} | -0.614 | 60h_mean_length_width_ratio | -0.276 |
| 18h_mean_length* | -1.757 | 18h_sd_length* | -0.917 | 18h_sd_length* | -0.701 | 2dh_sd_length | -0.440 |

| | | | | | | | |
|---|---|---|---|---|---|---|---|
| *Blue: Commonly used effective descriptor shared in models using small to large data-size. **Red: Commonly used effective descriptor shared in models using larger data-size. | | | | | | | |

"Correlation" and "Energy" are both texture descriptors; therefore, because they alter intensity profiles under phase-contrast microscopy, they generally reflect the three-dimensional patterns and complexity of cells. In practice, intensity profiles change dramatically when cells alter their three-dimensional roundness during cell division. Thus, an increase in "SD of texture" indicates the presence of a larger number of proliferating cells. Therefore, when "mean texture" has a greater influence, the cell population becomes more uniform in texture, meaning that the number of cellular events that alter texture decreases.

Length and compactness are shape-related descriptors and therefore generally reflect two-dimensional responses such as elongation and expansion. When such shape changes occur with little accompanying texture change, it indicates that cellular activity is being used more for elongation than for proliferation. Thus, information derived from such model structures suggests that the acquired serial passage prediction model is useful not only for early detection of future cell performance but also for quantitative extraction of morphologically descriptive rules. Accordingly, such descriptive understanding of morphological profiles helps move away from the old practice of grasping morphological changes by intuition.

When the same condition matrix was evaluated using the nonlinear machine learning model RF, this model did not outperform LASSO under any data usage condition (Fig. 4d). These data reflect that serial passage potency and morphological information have a linear relationship.

Although the inventors examined combinations of two parameters (time window and number of FOVs), model training is ultimately based on raw sample data that are relatively small in size compared with machine learning applications in other fields. Therefore, the inventors attempted to further examine the prediction performance of the LASSO model in more detail.

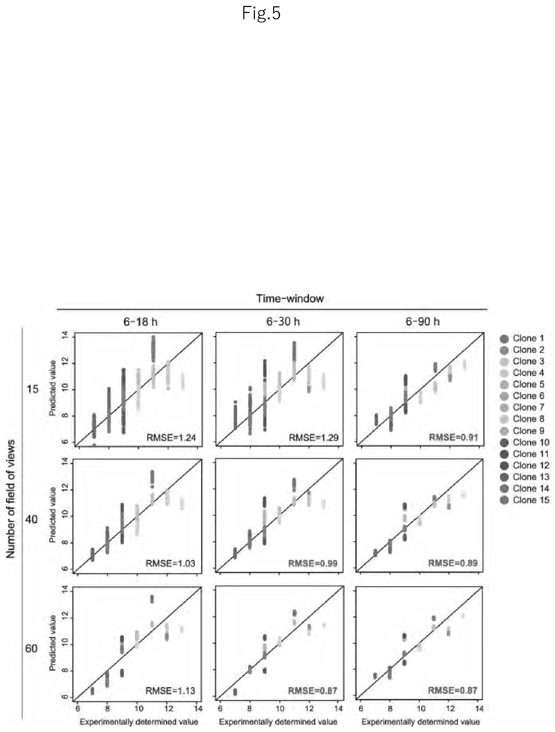

To evaluate how robust the prediction model is to variation in data, bootstrap resampling was introduced to increase variation in image-derived morphological profiles. By introducing 50 bootstraps and collecting various combinations of FOVs from 64 tiled images per sample, the performance of the prediction model was evaluated (Fig. 5).

These results indicate that such image sampling bias introduced by bootstrapping caused performance degradation in some prediction models. However, although the range of "time window effect" and "FOV number effect" required to achieve high-performance models (RMSE < 1.0) became narrower due to more robust models compared with Figs. 4a-4c, the earliest prediction remained achievable with FOV = 40 and a 6-30-hour window, and the minimum-effort prediction remained achievable with FOV = 15 and a 6-90-hour window. Thus, prediction accuracy (RMSE < 1.0) was maintained while minimizing the scale of data collection.

When evaluating the effect of such bootstrapping in PCA, the number of FOVs clearly improved the robustness of morphological profiles. Accumulation of morphological profiles over longer time windows improved the ability to distinguish differences among clones. Such investigation of data size effects contributes to designing more effective processes for introducing image-based quality checks in cell bank construction.

### Discussion

REC are clonal MSCs selected from human BMMCs, and they not only retain the superior qualities of conventionally processed MSCs but also possess characteristics advantageous for practical cell manufacturing processes for therapeutic products. In particular, the high proliferation potential of REC is a major advantage in developing efficient manufacturing processes for cell therapy products. Therefore, in this study, the possibility of predicting serial passage performance from early morphological information alone, as well as the most practical method for constructing such a prediction system, was examined so that REC performance can be evaluated from the earliest stages of cell bank construction.

In any type of cell culture, a long-standing unresolved issue is determining the "optimal timing for creating cryostocks" during expansion culture. Because most normal cells lose proliferation potential when cultured in vitro [38, 39], one must essentially gamble on which passage number will be the last while creating cryostocks. In cell bank manufacturing, such uncertainty represents a major risk. If cells fail to proliferate as expected during low-success-rate expansion culture, substantial costs are incurred; conversely, if cryopreservation of cells is performed too early, the resulting stock will not achieve sufficient production efficiency.

In practice, the bottleneck in actual cell bank construction is the effort required to recruit valuable donors, not the effort required for greedy selection of candidate cell banks. However, for REC, a more rigorous and selective process is expected to complete candidate cell banks as "master cell banks," and our study presents a new concept of using morphological non-invasive analysis as an "in-process analytical tool" to strengthen and optimize the cell bank construction process. This concept helps balance "cell yield" and "maintenance of banked-cell performance," and by predicting future serial passage potency, it assists in determining the optimal timing for creating cryostocks. Such an approach helps move away from the current cell bank design concept that limits passage numbers using data-less logic.

The inventors examined methods for predicting the "serial passage potency" of REC, but serial passage potency in cMSC may require some discussion. For induced pluripotent stem cells (iPSCs), it is understood that their uncontrolled proliferation potential can adversely affect therapy (e.g., risk of teratoma formation) [40, 41]. However, for MSCs, although their proliferation potential is known to be finite, their serial passage potency must be considered in several contexts.

If the "candidate cell bank" in this study is created as a master cell bank for producing further working cell banks, its serial passage potency would be beneficial for the entire process. However, if it is created as the final cell bank for transplantation, the effect of serial passage potency should be carefully examined. It may be advantageous when enhancing the efficacy of the final product, but if it adversely affects efficacy, it becomes a risk.

In any case, performing such future performance prediction from the earliest stages of the process helps optimize the quality of the final cell bank. This is because such prediction can only be evaluated through excessive continuous evaluation of unproductive REC candidates. Since REC are currently progressing toward clinical trials, our next challenge is to validate the effectiveness of such performance prediction and efficiently advance product manufacturing.

Finally, morphology-based prediction of future REC performance raises the question of whether this developed model can be applied to studies establishing other MSC cell banks. The inventors' detailed image-based morphological measurements showed that the major REC population consists of cells nearly twice as small as bulk MSCs. Such a large size difference makes the prediction model structure, based on combinations of morphological descriptors, well suited to REC, because the inventors used "mean and SD" to reflect cell population distributions in morphological profiles.

### [EXAMPLE 2]

In Example 2, comparisons of population distributions of entire cell populations were performed.

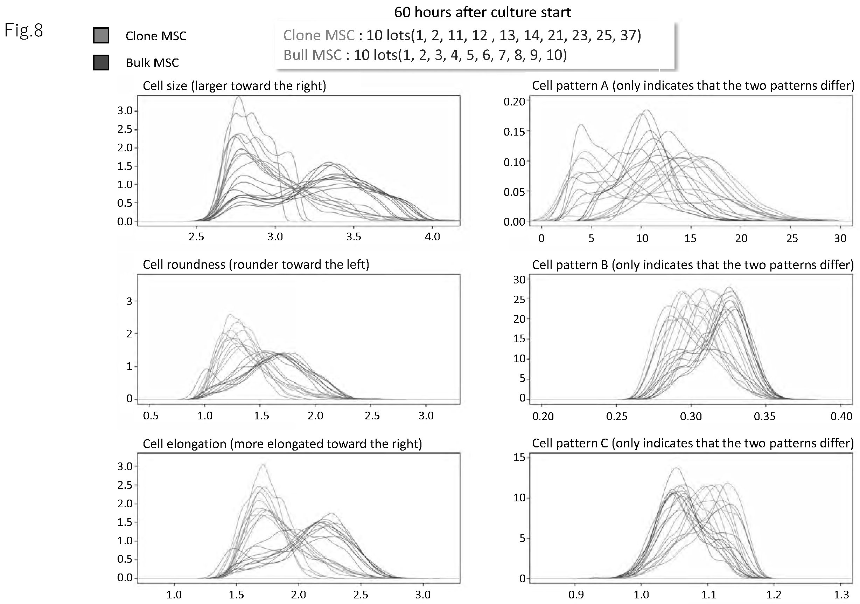

The results are shown in Figs. 8-16.

Fig. 8 shows the results of comparing population distributions of entire cell populations. Clone MSCs (rapidly expanding mesenchymal stem cells characterized by using, as an indicator, the positivity or negativity of at least one marker selected from LNGFR (CD271) and Thy-1 (CD90)) and bulk MSCs (mesenchymal stem cells not characterized by these markers) were cultured. Phase-contrast microscopic images obtained from culture vessels were processed to describe population distributions consisting of several thousand cells within each field of view. The six panels represent six types of morphological features, and the population distributions were plotted using each one-dimensional feature. The results showed that clone MSCs and bulk MSCs exhibited markedly different population distributions for all morphological features. Compared with Fig. 3, the image quality, time points used for comparison, and image processing methods differed, and measurements were obtained from more stable cell image data.

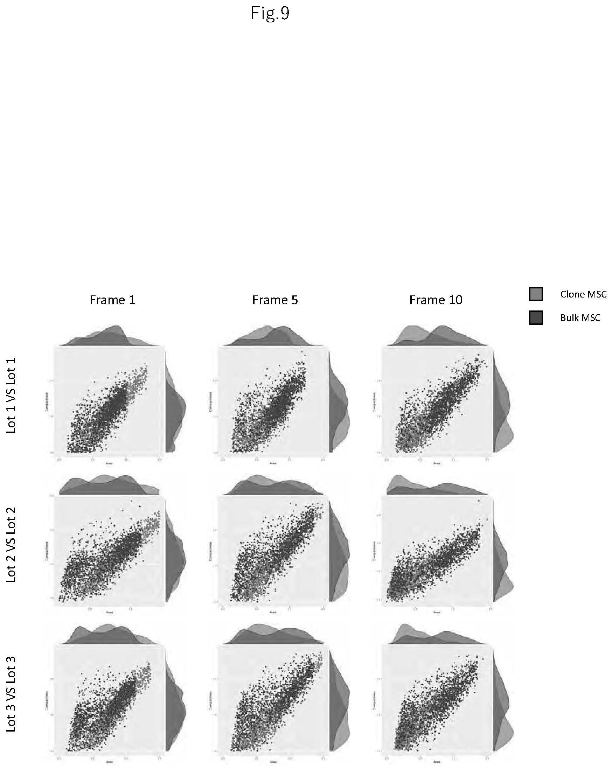

Fig. 9 shows the results of comparing population distributions of entire cell populations. Clone MSCs (rapidly expanding mesenchymal stem cells characterized by using, as an indicator, the positivity or negativity of at least one marker selected from LNGFR (CD271) and Thy-1 (CD90)) and bulk MSCs (mesenchymal stem cells not characterized by these markers) were cultured (three lots each). Phase-contrast microscopic images obtained from culture vessels were processed to describe population distributions consisting of tens of thousands of cells within each field of view, not as single-feature distributions but as two-feature combination scatter plots. Fig. 9 shows differences between lots (row differences) and differences in time-series data (column differences). By observing two-dimensional combinations (two features), it was confirmed that bulk MSCs contain multiple subpopulations (multiple distributions), when compared with clone MSCs. Compared with Fig. 3, the image quality, time points used for comparison, and image processing methods differed, and measurements were obtained from more stable cell image data.

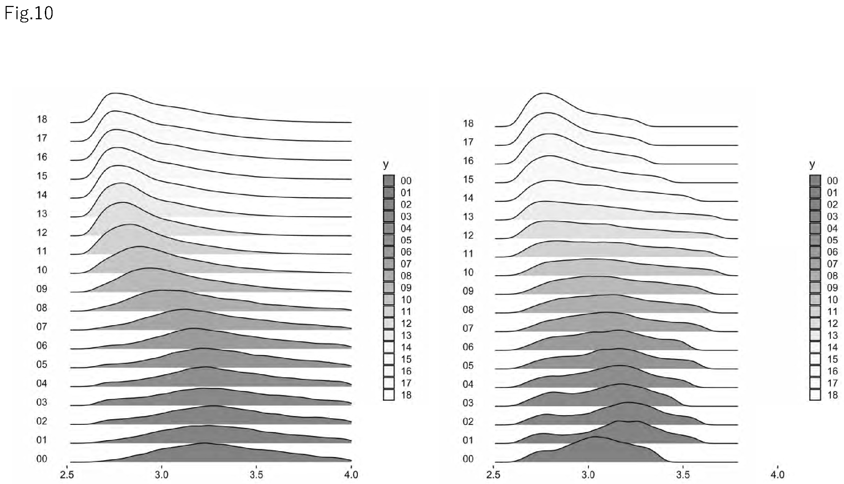

Fig. 10 shows the results of comparing population distributions of entire cell populations. Clone MSCs (rapidly expanding mesenchymal stem cells characterized by using, as an indicator, the positivity or negativity of at least one marker selected from LNGFR (CD271) and Thy-1 (CD90)) and bulk MSCs (mesenchymal stem cells not characterized by these markers) were cultured. Phase-contrast microscopic images obtained from culture vessels were processed to show time-dependent changes in population distributions consisting of tens of thousands of cells within each field of view. The horizontal axis represents cell size. When comparing time-series data, differences were observed between clone MSCs (left) and bulk MSCs (right) in terms of the diversity of distributions and the timing at which such diversity appeared. The y axis represents the time series (6-hour intervals). Clone MSCs (REC) maintained a single distribution over time (higher rows represent later culture stages), whereas bulk MSCs (commercially available MSCs) initially exhibited two peaks, which then merged or mixed over time. This indicates that "the cell population is complex and contains multiple subpopulations."

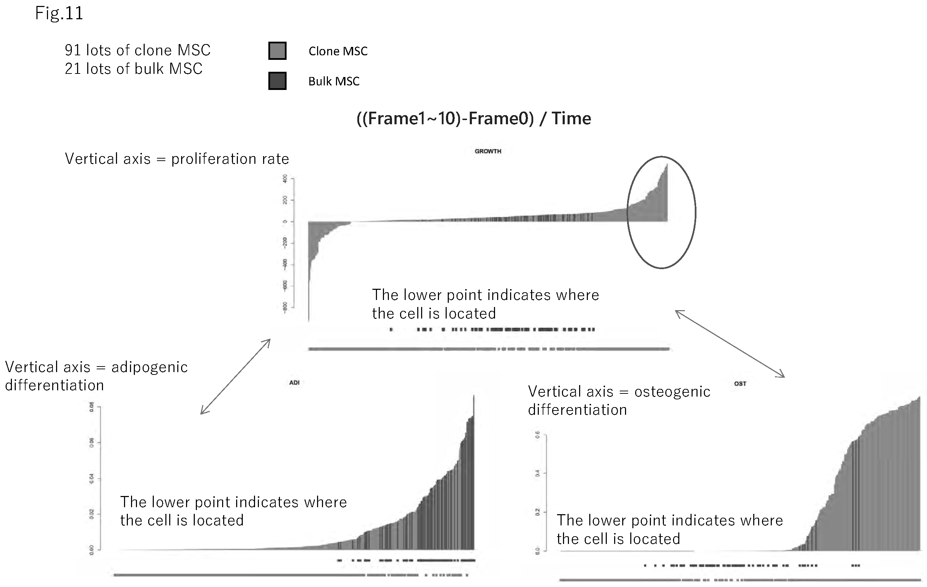

Fig. 11 shows the results of evaluating proliferation rate, adipogenic differentiation, and osteogenic differentiation of each MSC.

Clone MSCs (rapidly expanding mesenchymal stem cells characterized by using, as an indicator, the positivity or negativity of at least one marker selected from LNGFR (CD271) and Thy-1 (CD90)) from 91 lots and bulk MSCs (mesenchymal stem cells not characterized by these markers) from 21 lots were cultured. The following evaluations were performed: (i) proliferation rate over 7 days of culture, (ii) adipogenic differentiation after 2 weeks, and (iii) osteogenic differentiation after 2 weeks. Proliferation rate was determined by counting cells using image processing of time-series images. For adipogenic differentiation, cells were cultured in adipogenic differentiation induction medium for 2 weeks, stained with Oil Red O, and the differentiation potential score was calculated as the total area of red-stained regions in the images. For osteogenic differentiation, cells were cultured in osteogenic differentiation induction medium for 2 weeks, stained with Alizarin Red S, and the differentiation potential score was calculated as the total area of red-stained regions in the images. All results were aggregated and displayed in ascending order for (i), (ii), and (iii). The results showed that clone MSCs without strong selection exhibited a large amount of data showing a wide variety of qualities (proliferation and differentiation potentials), whereas bulk MSCs, even when purchased in multiple lots for a wide variety of variations, showed very limited diversity (blue bars show bias in quality values they represent).

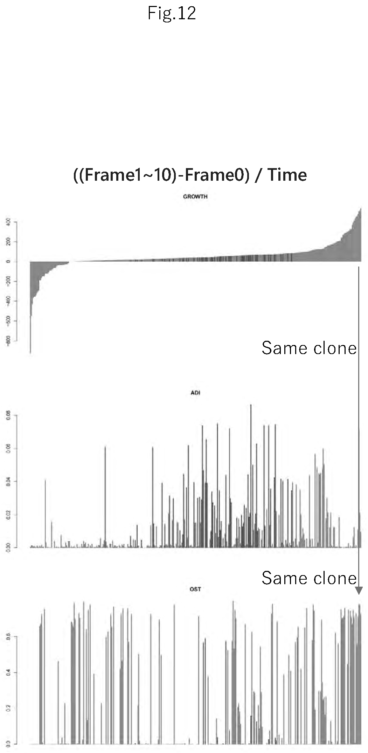

Fig. 12 displays the adipogenic and osteogenic differentiation quality scores as bar graphs, arranged in the same ascending order of proliferation rate as in Fig. 11. Fig. 12 shows that there is no simple correlation such as "high proliferation rate = high differentiation" or "low proliferation rate = low differentiation," nor does high proliferation rate strongly influence either adipogenic or osteogenic differentiation. Thus, to prepare a wide range of cell qualities, it is effective to accumulate diverse clones; collecting only fast-proliferating clones does not cover all qualities. Furthermore, because the quality of purchased cells ((i), (ii), (iii)) generally cannot be known beforehand, it is impossible to assemble data variation using only pre-acquired information. Therefore, clone MSCs are effective for obtaining data variation. Additionally, clone MSCs cover a much wider range of qualities than bulk MSCs. In particular, proliferation potential in clone MSCs far exceeds the range of qualities achievable by bulk MSCs.

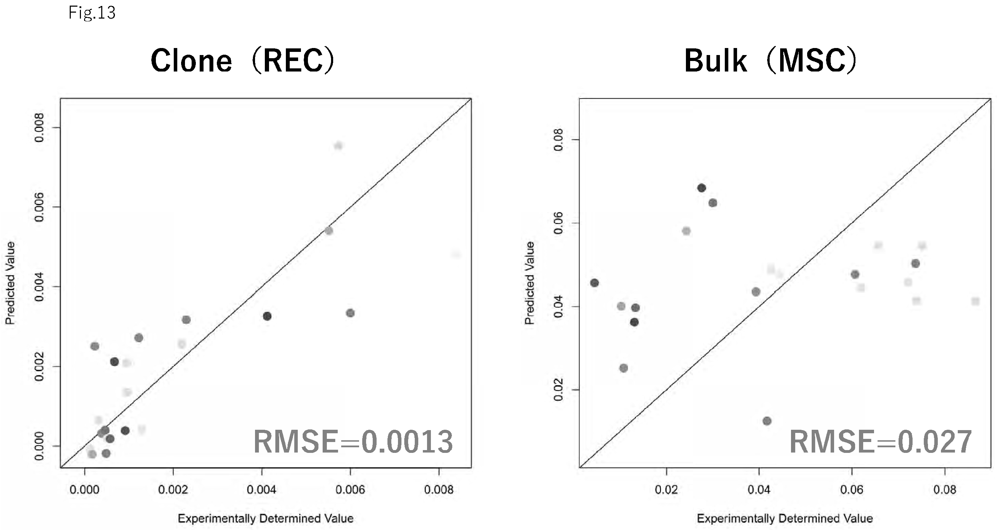

Fig. 13 shows proliferation rates of each MSC measured by time-series image analysis.

Clone MSCs (rapidly expanding mesenchymal stem cells characterized by using, as an indicator, the positivity or negativity of at least one marker selected from LNGFR (CD271) and Thy-1 (CD90)) from 20 lots and bulk MSCs (mesenchymal stem cells not characterized by these markers) from 20 lots were cultured. Proliferation rates (day 1-day 5) were measured by time-series image analysis (actual values plotted on the horizontal axis). Then, using each cell lot as one sample, a machine learning model was constructed using a dataset, with:
- explanatory variables: cell morphological information for each sample (360 parameters = population statistics (mean and SD: 2 types) of 12 morphological descriptors for several thousand cells × 15 time points (90 hours)); and
- objective variable: proliferation rate.
The model predicted proliferation rate on day 5. Fig. 13 shows that machine learning models for the proliferation rate prediction model, constructed using clone MSC data, had smaller errors (lower RMSE), demonstrating the effectiveness of using clone MSC data for machine learning model construction.

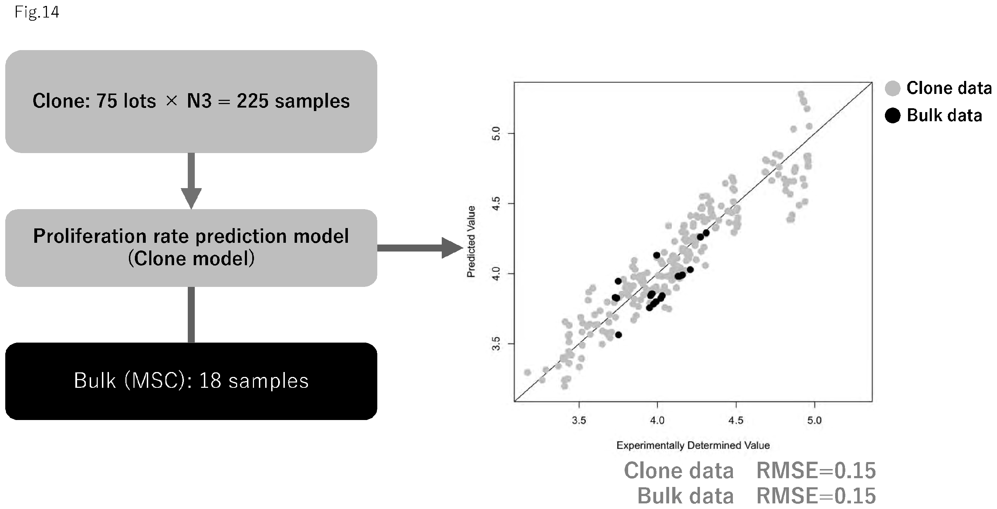

Fig. 14 shows proliferation rates of each MSC measured by time-series image analysis.

Clone MSCs (rapidly expanding mesenchymal stem cells characterized by using, as an indicator, the positivity or negativity of at least one marker selected from LNGFR (CD271) and Thy-1 (CD90)) from 75 samples and bulk MSCs (mesenchymal stem cells not characterized by these markers) from 18 lots were cultured. Proliferation rates (day 1-day 5) were measured by time-series image analysis (actual values plotted on the horizontal axis). Then, using only clone MSC data, a machine learning model was constructed using a dataset, with:
- explanatory variables: cell morphological information for each sample (360 parameters = population statistics (mean and SD: 2 types) of 12 morphological descriptors for several thousand cells × 15 time points (90 hours)); and
- objective variable: proliferation rate.
The model predicted proliferation rate on day 5 (gray dots in the scatter plot). Then, the explanatory variables, i.e., 360 morphological parameters, obtained from bulk MSCs were input into the proliferation rate prediction model constructed using clone MSCs. The results (black dots in the scatter plot) showed that proliferation rates of bulk MSC samples, never used in training and not belonging to clone MSCs, could be predicted with high accuracy using morphological information alone.

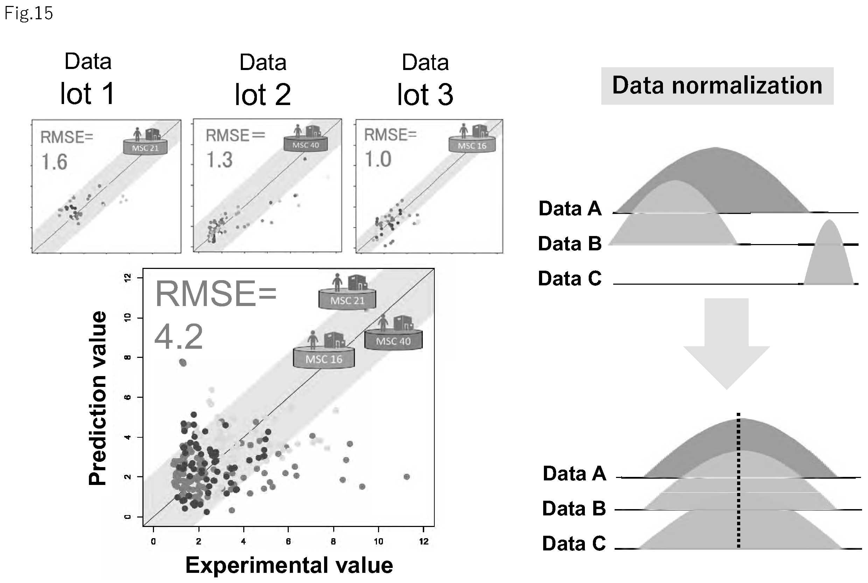

Fig. 15 shows proliferation rates of each MSC measured by time-series image analysis.

Clone MSCs (rapidly expanding mesenchymal stem cells characterized by using, as an indicator, the positivity or negativity of at least one marker selected from LNGFR (CD271) and Thy-1 (CD90)) were cultured (different lots for each experiment, totaling 75 culture lots). Proliferation rates (day 1-day 5) were measured by time-series image analysis (actual values plotted on the horizontal axis). For each lot (different experiment days), a machine learning model was constructed using:
- explanatory variables: morphological information (360 parameters = population statistics (mean and SD: 2 types) of 12 morphological descriptors for several thousand cells × 15 time points (90 hours)); and
- objective variable: proliferation rate.
Low prediction errors (low RMSE) were obtained for each experiment day (upper panel). However, when all data were simply combined and a single proliferation prediction model was constructed using all 75 lots, prediction error deteriorated significantly. This indicates that even when all images are captured using the same automated culture observation system, data bias exists between experiment days, and machine learning models built from naively combined data perform poorly.

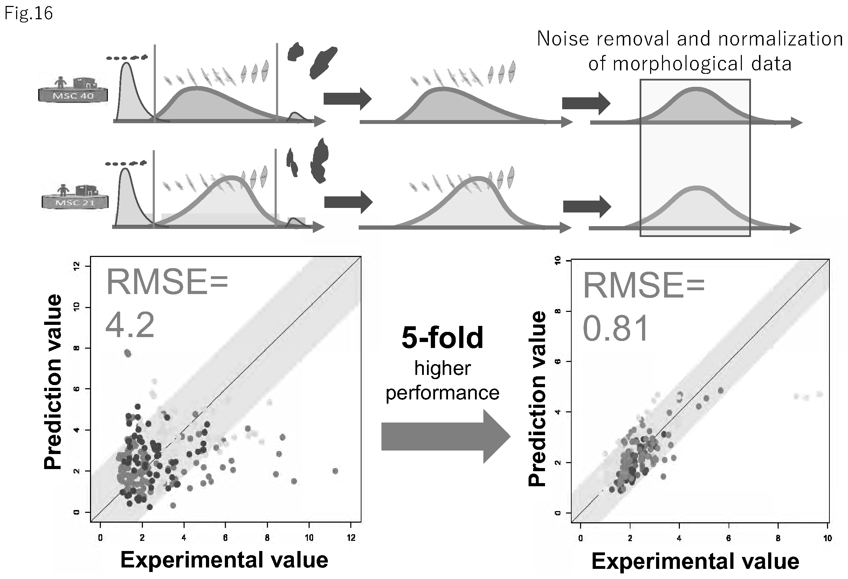

Fig. 16 shows the results of data integration. In the data integration of Fig. 15, preprocessing combining data curation, noise removal, and data normalization was applied to each dataset. The results showed that prediction errors (RMSE) of machine learning models were lower and more stable when such preprocessing was applied (right panel) compared with when it was not applied (left panel). This demonstrates that even when all images are captured using the same automated culture observation system, data bias exists between experiment days, and machine learning models built from naively combined data perform poorly.

### References

1. Marofi F, Vahedi G, Biglari A, Esmaeilzadeh A, Athari SS. Mesenchymal stromal/stem cells: a new era in the cell-based targeted gene therapy of cancer. Front Immunol. 2017;8:1770. https:// doi. org/ 10. 3389/ fimmu. 2017.01770.
2. Sherman LS, Shaker M, Mariotti V, Rameshwar P. Mesenchymal stromal/stem cells in drug therapy: new perspective. Cytotherapy. 2017;19(1):19-27. https:// doi. org/ 10. 1016/j. jcyt. 2016. 09. 007.
3. Squillaro T, Peluso G, Galderisi U. Clinical trials with mesenchymal stem cells: an update. Cell Transplant. 2016;25(5):829-48. https:// doi. org/ 10. 3727/ 09636 8915X 689622.
4. Wakao S, Kitada M, Kuroda Y, Shigemoto T, Matsuse D, Akashi H, Tanimura Y, Tsuchiyama K, Kikuchi T, Goda M, Nakahata T, Fujiyoshi Y, Dezawa M. Multilineage-differentiating stress-enduring (Muse) cells are a primary source of induced pluripotent stem cells in human fibroblasts. Proc Natl Acad Sci USA. 2011;108(24):9875-80. https:// doi. org/ 10. 1073/ pnas. 11008 16108.
5. Vogel W, Grunebach F, Messam CA, Kanz L, Brugger W, Buhring HJ. Heterogeneity among human bone marrow-derived mesenchymal stem cells and neural progenitor cells. Haematologica. 2003;88(2):126-33.
6. Trivanovic D, Jaukovic A, Popovic B, Krstic J, Mojsilovic S, Okic-Djordjevic I, Kukolj T, Obradovic H, Santibanez JF, Bugarski D. Mesenchymal stem cells of different origin: comparative evaluation of proliferative capacity, telomere length and pluripotency marker expression. Life Sci. 2015;141:61-73. https:// doi. org/ 10. 1016/j. lfs. 2015. 09. 019.
7. Sacchetti B, Funari A, Remoli C, et al. No identical "mesenchymal stem cells" at different times and sites: human committed progenitors of distinct origin and differentiation potential are incorporated as adventitial cells in microvessels. Stem Cell Reports. 2016;6(6):897-913. https:// doi. org/ 10. 1016/j. stemcr. 2016. 05. 011.
8. Bakopoulou A, Apatzidou D, Aggelidou E, Gousopoulou E, Leyhausen G, Volk J, Kritis A, Koidis P, Geurtsen W. Isolation and prolonged expansion of oral mesenchymal stem cells under clinical-grade, GMP-compliant conditions differentially affects "stemness" properties. Stem Cell Res Ther. 2017;8(1):247. https:// doi. org/ 10. 1186/ s13287- 017- 0705-0.
9. Colter DC, Class R, DiGirolamo CM, Prockop DJ. Rapid expansion of recycling stem cells in cultures of plastic-adherent cells from human bone marrow. Proc Natl Acad Sci USA. 2000;97(7):3213-8. https:// doi. org/ 10. 1073/ pnas. 97.7. 3213.
10. Mabuchi Y, Morikawa S, Harada S, Niibe K, Suzuki S, Renault-Mihara F, Houlihan DD, Akazawa C, Okano H, Matsuzaki Y. LNGFR+THY-1+VCAM-1hi+ cells reveal functionally distinct subpopulations in mesenchymal stem cells. Stem Cell Reports. 2013;1(2):152-65. https:// doi. org/ 10. 1016/j. stemcr. 2013. 06. 001.
11. Ukeba D, Yamada K, Tsujimoto T, Ura K, Nonoyama T, Iwasaki N, Sudo H. Bone marrow aspirate concentrate combined with in situ forming bioresorbable gel enhances intervertebral disc regeneration in rabbits. J Bone Joint Surg. 2021; 103(8): e31. https:// doi. org/ 10. 2106/ JBJS. 20. 00606.
12. De Bari C, Roelofs AJ. Stem cell-based therapeutic strategies for cartilage defects and osteoarthritis. Curr Opin Pharmacol. 2018;40:74-80. https://doi. org/ 10. 1016/j. coph. 2018. 03. 009.
13. Kabat M, Bobkov I, Kumar S, Grumet M. Trends in mesenchymal stem cell clinical trials 2004-2018: is efficacy optimal in a narrow dose range? Stem Cells Transl Med. 2020;9(1):17-27. https:// doi. org/ 10. 1002/ sctm. 19- 0202.
14. Viswanathan S, Keating A, Deans R, Hematti P, Prockop D, Stroncek DF, Stacey G, Weiss DJ, Mason C, Rao MS. Soliciting strategies for developing cell-based reference materials to advance mesenchymal stromal cell research and clinical translation. Stem Cells and Development. 2014;23(11):1157-67. https:// doi. org/ 10. 1089/ scd. 2013. 0591.
15. Dwarshuis NJ, Parratt K, Santiago-Miranda A, Roy K. Cells as advanced therapeutics: state-of-the-art, challenges, and opportunities in large scale biomanufacturing of high-quality cells for adoptive immunotherapies. Adv Drug Deliv Rev. 2017;114:222-39. https:// doi. org/ 10. 1016/j. addr. 2017. 06. 005.
16. Jossen V, van den Bos C, Eibl R, Eibl D. Manufacturing human mesenchymal stem cells at clinical scale: process and regulatory challenges. Appl Microbiol Biotechnol. 2018;102:3981-94. https:// doi. org/ 10. 1007/ s00253- 018- 8912-x.
17. Lo Surdo J, Bauer SR. Quantitative approaches to detect donor and passage differences in adipogenic potential and clonogenicity in human bone marrow-derived mesenchymal stem cells. Tissue Engineering - Part C: Methods. 2012;18(11):877-89. https:// doi. org/ 10. 1089/ ten. tec. 2011. 0736.
18. Mohamed-Ahmed S, Fristad I, Lie SA, Suliman S, Mustafa K, Vindenes H, Idris SB. Adipose-derived and bone marrow mesenchymal stem cells: a donor-matched comparison. Stem Cell Res Ther. 2018;9(1):168. https:// doi. org/ 10. 1186/ s13287- 018- 0914-1.
19. Fennema EM, Renard AJS, Leusink A, Van Blitterswijk CA, De Boer J. The effect of bone marrow aspiration strategy on the yield and quality of human mesenchymal stem cells. Acta Orthop. 2009;80(5):618-21. https://doi. org/ 10. 3109/ 17453 67090 32782 41.
20. Collart-Dutilleul P-Y, Chaubron F, De Vos J, Cuisinier FJ. Allogenic banking of dental pulp stem cells for innovative therapeutics. World Journal of Stem Cells. 2015;7(7): 1010-21. https:// doi. org/ 10. 4252/ wjsc. v7. i7. 1010.
21. Nievaleve S. Concise review: umbilical cord derived mesenchymal stem cell bank. Progress in Stem Cell. 2017;4:3-4. https:// doi. org/ 10. 15419/ psc. v4i3-4. 397.
22. Alzahrani FA, Saadeldin IM, Ahmad A, Kumar D, Azhar EI, Siddiqui AJ, Kurdi B, Sajini A, Alrefaei AF, Jahan S. The potential use of mesenchymal stem cells and their derived exosomes as immunomodulatory agents for COVID-19 patients. Stem Cells International. 2020;2020:8835986. https:// doi. org/ 10. 1155/ 2020/ 88359 86.
23. Rombouts WJC, Ploemacher RE. Primary murine MSC show highly efficient homing to the bone marrow but lose homing ability following culture. Leukemia. 2003;17(1):160-70. https:// doi. org/ 10. 1038/ sj. leu. 24027 63.
24. Dhanasekaran M, Indumathi S, Poojitha R, Kanmani A, Rajkumar JS, Sudarsanam D. Plasticity and banking potential of cultured adipose tissue derived mesenchymal stem cells. Cell Tissue Banking. 2013;14(2):303-15. https:// doi. org/ 10. 1007/ s10561- 012- 9311-7.
25. Dave C, McRae A, Doxtator E, Mei SHJ, Sullivan K, Wolfe D, Champagne J, McIntyre L. Comparison of freshly cultured versus freshly thawed (cryopreserved) mesenchymal stem cells in preclinical in vivo models of inflammation: a protocol for a preclinical systematic review and meta-analysis. Syst Rev. 2020;9(1):188. https:// doi. org/ 10. 1186/ s13643- 020-01437-z.
26. Hladik D, Hofig I, Oestreicher U, Beckers J, Matjanovski M, Bao X, Scherthan H, Atkinson MJ, Rosemann M. Long-term culture of mesenchymal stem cells impairs ATM-dependent recognition of DNA breaks and increases genetic instability. Stem Cell Res Ther. 2019;10(1):218. https:// doi. org/ 10. 1186/ s13287- 019- 1334-6.
27. Jin HJ, Bae YK, Kim M, Kwon SJ, Jeon HB, Choi SJ, Kim SW, Yang YS, Oh W, Chang JW. Comparative analysis of human mesenchymal stem cells from bone marrow, adipose tissue, and umbilical cord blood as sources of cell therapy. Int J Mol Sci. 2013;14(9):218. https:// doi. org/ 10. 3390/ ijms1 40917 986.
28. Yang YHK, Ogando CR, Wang See C, Chang TY, Barabino GA. Changes in phenotype and differentiation potential of human mesenchymal stem cells aging in vitro. Stem Cell Res Ther. 2018;9(1):131. https:// doi. org/ 10. 1186/ s13287- 018- 0876-3.
29. Wagner W, Horn P, Castoldi M, Diehlmann A, Bork S, Saffrich R, Benes V, Blake J, Pfister S, Eckstein V, Ho AD. Replicative senescence of mesenchymal stem cells: a continuous and organized process. PLoS ONE. 2008;3(5): e2213. https:// doi. org/ 10. 1371/ journ al. pone. 00022 13.
30. Turinetto V, Vitale E, Giachino C. Senescence in human mesenchymal stem cells: functional changes and implications in stem cell-based therapy. Int J Mol Sci. 2016;17(7):1164. https:// doi. org/ 10. 3390/ ijms1 70711 64.
31. Binato R, de Souza FT, Lazzarotto-Silva C, Du Rocher B, Mencalha A, Pizzatti L, Bouzas LF, Abdelhay E. Stability of human mesenchymal stem cells during in vitro culture: considerations for cell therapy. Cell Prolif. 2013;46(1):10-22. https:// doi. org/ 10. 1111/ cpr. 12002.
32. Galipeau J, Sensebe L. Mesenchymal stromal cells: clinical challenges and therapeutic opportunities. Cell Stem Cell. 2018;22(6):824-33. https:// doi. org/ 10. 1016/j. stem. 2018. 05. 004.
33. Samsonraj RM, Raghunath M, Nurcombe V, Hui JH, van Wijnen AJ, Cool SM. Concise review: multifaceted characterization of human mesenchymal stem cells for use in regenerative medicine. Stem Cells Transl Med. 2017;6(12):2173-85. https:// doi. org/ 10. 1002/ sctm. 17- 0129.
34. Imai Y, Yoshida K, Matsumoto M, Okada M, Kanie K, Shimizu K, Honda H, Kato R. In-process evaluation of culture errors using morphology-based image analysis. Regenerative Therapy. 2018;9(9):15-23. https:// doi. org/ 10. 1016/j. reth. 2018. 06. 001.
35. Matsuoka F, Takeuchi I, Agata H, Kagami H, Shiono H, Kiyota Y, Honda H, Kato R. Morphology-based prediction of osteogenic differentiation potential of human mesenchymal stem cells. PLoS ONE. 2013;8(2): e55082. https:// doi. org/ 10. 1371/ journ al. pone. 00550 82.
36. Sasaki H, Takeuchi I, Okada M, Sawada R, Kanie K, Kiyota Y, Honda H, Kato R. Label-free morphology-based prediction of multiple differentiation potentials of human mesenchymal stem cells for early evaluation of intact cells. PLoS ONE. 2014;9(4): e93952. https:// doi. org/ 10. 1371/ journ al. pone. 00939 52.
37. Takemoto Y, Imai Y, Kanie K, Kato R. Predicting quality decay in continuously passaged mesenchymal stem cells by detecting morphological anomalies. J Biosci Bioeng. 2020;131(2):198-206. https:// doi. org/ 10. 1016/j. jbiosc. 2020. 09. 022.
38. Sawada R, Ito T, Tsuchiya T. Changes in expression of genes related to cell proliferation in human mesenchymal stem cells during in vitro culture in comparison with cancer cells. J Artif Organs. 2006;9(3):179-84. https:// doi. org/ 10. 1007/ s10047- 006- 0338-z.
39. Muraglia A, Cancedda R, Quarto R. Clonal mesenchymal progenitors from human bone marrow differentiate in vitro according to a hierarchical model. J Cell Sci. 2000;113(7):1161-6. https:// doi. org/ 10. 1242/ jcs. 113.7. 1161.
40. Okita K, Ichisaka T, Yamanaka S. Generation of germline-competent induced pluripotent stem cells. Nature. 2007;448(7151):313-7. https:// doi. org/ 10. 1038/ natur e05934.
41. Gutierrez-Aranda I, Ramos-Mejia V, Bueno C, Munoz-Lopez M, Real PJ, Macia A, Sanchez L, Ligero G, Garcia-Parez JL, Menendez P. Human induced pluripotent stem cells develop teratoma more efficiently and faster than human embryonic stem cells regardless the site of injection. Stem Cells. 2010;28(9):1568-70. https:// doi. org/ 10. 1002/ stem. 471.

## Claims

1. A reference standard for evaluating or determining the quality of cells of interest, the reference standard comprising a rapidly expanding mesenchymal stem cell population **characterized by** using, as an indicator, the positivity or negativity of at least one marker selected from LNGFR (CD271) and Thy-1 (CD90).

2. The reference standard according to claim 1, wherein characteristics of the cell population are selected from any of the following information (a) to (d):
(a) at least one piece of cell information selected from the group consisting of cell size, degree of spreading, contour complexity, pattern, and brightness of individual cells within the cell population, and image information thereof;
(b) information obtained by integrating the cell information of (a) across multiple cells within the cell population and statistically processing it;
(c) combination information obtained by acquiring the information of (b) for multiple cell populations; and
(d) information of (a) to (c) acquired over time.

3. The reference standard according to claim 2, wherein the statistically processed information is at least one selected from the group consisting of mean value, standard deviation, and distribution shape.

4. The reference standard according to claim 1, wherein the quality is at least one selected from the group consisting of cell proliferation rate, cell recovery number, degree of undifferentiation, degree of differentiation, amount of produced factors, and cell heterogeneity.

5. The reference standard according to claim 1, wherein the cells of interest are stem cells.

6. The reference standard according to claim 5, wherein the stem cells of interest are mesenchymal stem cells.

7. A method for providing information for evaluating the quality of a stem cell population of interest, the method comprising the steps of:
(a) characterizing individual cells of a rapidly expanding mesenchymal stem cell population by using, as an indicator, the positivity or negativity of at least one marker selected from LNGFR (CD271) and Thy-1 (CD90), and acquiring image data (reference image information) of the individual cells, or acquiring parameter information (reference parameter information) representing characteristics of the cell population from the reference image information;
(b) acquiring image data (image information of interest) of individual cells of a stem cell population of interest provided by a user, or acquiring parameter information (parameter information of interest) representing characteristics of the stem cell population of interest from the image information of interest;
(c) comparing the image information of interest or parameter information of interest with the reference image information or reference parameter information; and
(d) providing to the user all or part of the information obtained by the comparison for evaluating the quality of the stem cell population of interest.

8. A method for evaluating the quality of a stem cell population of interest, the method comprising the steps of:
(a) characterizing individual cells of a rapidly expanding mesenchymal stem cell population by using, as an indicator, the positivity or negativity of at least one marker selected from LNGFR (CD271) and Thy-1 (CD90), and acquiring image data (reference image information) of the individual cells, or acquiring parameter information (reference parameter information) representing characteristics of the cell population from the reference image information;
(b) acquiring image data (image information of interest) of individual cells of a stem cell population of interest provided by a user, or acquiring parameter information (parameter information of interest) representing characteristics of the stem cell population of interest from the image information of interest;
(c) comparing the image information of interest or parameter information of interest with the reference image information or reference parameter information; and
(d) evaluating the quality of stem cells of interest by using, as an indicator, all or part of the information obtained by the comparison.

9. A system for providing information for evaluating the quality of a stem cell population of interest, the system comprising:
(a) a unit configured to characterize individual cells of a rapidly expanding mesenchymal stem cell population by using, as an indicator, the positivity or negativity of at least one marker selected from LNGFR (CD271) and Thy-1 (CD90), and acquire image data (reference image information) of the individual cells, or acquire parameter information (reference parameter information) representing characteristics of the cell population from the reference image information;
(b) a unit configured to acquire image data (image information of interest) of individual cells of a stem cell population of interest provided by a user, or acquire parameter information (parameter information of interest) representing characteristics of the stem cell population of interest from the image information of interest;
(c) a unit configured to compare the image information of interest or parameter information of interest with the reference image information or reference parameter information; and
(d) a unit configured to provide to the user all or part of the information obtained by the comparison for evaluating the quality of the stem cell population of interest.

10. A system for evaluating the quality of a stem cell population of interest, the system comprising:
(a) a unit configured to characterize individual cells of a rapidly expanding mesenchymal stem cell population by using, as an indicator, the positivity or negativity of at least one marker selected from LNGFR (CD271) and Thy-1 (CD90), and acquire image data (reference image information) of the individual cells, or acquire parameter information (reference parameter information) representing characteristics of the cell population from the reference image information;
(b) a unit configured to acquire image data (image information of interest) of individual cells of a stem cell population of interest provided by a user, or acquire parameter information (parameter information of interest) representing characteristics of the stem cell population of interest from the image information of interest;
(c) a unit configured to compare the image information of interest or parameter information of interest with the reference image information or reference parameter information; and
(d) a unit configured to evaluate the quality of stem cells of interest by using, as an indicator, all or part of the information obtained by the comparison.

11. The system according to claim 9 or 10, wherein characteristics of the cell population are selected from any of the following information (a) to (d):
(a) at least one piece of cell information selected from the group consisting of cell size, degree of spreading, contour complexity, pattern, and brightness of individual cells within the cell population, and image information thereof;
(b) information obtained by integrating the cell information of (a) across multiple cells within the cell population and statistically processing it;
(c) combination information obtained by acquiring the information of (b) for multiple cell populations; and
(d) information of (a) to (c) acquired over time.

12. The system according to claim 11, wherein the statistically processed information is at least one selected from the group consisting of mean value, standard deviation, and distribution shape.

13. The system according to claim 9 or 10, wherein the quality is at least one selected from the group consisting of cell proliferation rate, cell recovery number, degree of undifferentiation, degree of differentiation, amount of produced factors, and cell heterogeneity.

14. The system according to claim 9 or 10, wherein the stem cells of interest are mesenchymal stem cells.

15. A program for providing information for evaluating the quality of a stem cell population of interest, the program causing a computer to function as:
(a) a unit configured to characterize individual cells of a rapidly expanding mesenchymal stem cell population by using, as an indicator, the positivity or negativity of at least one marker selected from LNGFR (CD271) and Thy-1 (CD90), and acquire image data (reference image information) of the individual cells, or acquire parameter information (reference parameter information) representing characteristics of the cell population from the reference image information;
(b) a unit configured to acquire image data (image information of interest) of individual cells of a stem cell population of interest provided by a user, or acquire parameter information (parameter information of interest) representing characteristics of the stem cell population of interest from the image information of interest;
(c) a unit configured to compare the image information of interest or parameter information of interest with the reference image information or reference parameter information; and
(d) a unit configured to provide to the user all or part of the information obtained by the comparison for evaluating the quality of the stem cell population of interest.

16. A program for evaluating the quality of a stem cell population of interest, the program causing a computer to function as:
(a) a unit configured to characterize individual cells of a rapidly expanding mesenchymal stem cell population by using, as an indicator, the positivity or negativity of at least one marker selected from LNGFR (CD271) and Thy-1 (CD90), and acquire image data (reference image information) of the individual cells, or acquire parameter information (reference parameter information) representing characteristics of the cell population from the reference image information;
(b) a unit configured to acquire image data (image information of interest) of individual cells of a stem cell population of interest provided by a user, or acquire parameter information (parameter information of interest) representing characteristics of the stem cell population of interest from the image information of interest;
(c) a unit configured to compare the image information of interest or parameter information of interest with the reference image information or reference parameter information; and
(d) a unit configured to evaluate the quality of stem cells of interest by using, as an indicator, all or part of the information obtained by the comparison.

17. The program according to claim 15 or 16, wherein characteristics of the cell population are selected from any of the following information (a) to (d):
(a) at least one piece of cell information selected from the group consisting of cell size, degree of spreading, contour complexity, pattern, and brightness of individual cells within the cell population, and image information thereof;
(b) information obtained by integrating the cell information of (a) across multiple cells within the cell population and statistically processing it;
(c) combination information obtained by acquiring the information of (b) for multiple cell populations; and
(d) information of (a) to (c) acquired over time.

18. The program according to claim 17, wherein the statistically processed information is at least one selected from the group consisting of mean value, standard deviation, and distribution shape.

19. The program according to claim 15 or 16, wherein the quality is at least one selected from the group consisting of cell proliferation rate, cell recovery number, degree of undifferentiation, degree of differentiation, amount of produced factors, and cell heterogeneity.

20. The program according to claim 15 or 16, wherein the stem cells of interest are mesenchymal stem cells.

21. A computer-readable recording medium storing the program according to claim 15 or 16.

22. An image processing method for rapidly expanding mesenchymal stem cells, the method comprising the steps of:
(a) acquiring image data of the reference standard according to claim 1; and
(b) removing noise from the acquired image data and/or normalizing the image data.

23. A method for providing image data processed by the method according to claim 22 for machine learning by a computer.

24. A method for predicting the quality of cells of interest by comparing image data processed by the method according to claim 22 with image data of the cells of interest.
